# EUROPEAN PATENT SPECIFICATION

(11) **EP 3 717 908 B1**
(45) Date of publication and mention of the grant of the patent: **21.05.2025**
(21) Application number: 18882748.9
(22) Date of filing: 29.11.2018
(51) Int. Cl.: G01N 33/50, C12N 5/071, C12N 5/00, C12N 5/077

(54) **BIOREACTOR SCREENING PLATFORM FOR MODELLING HUMAN SYSTEMS BIOLOGY AND FOR SCREENING FOR INOTROPIC EFFECTS OF AGENTS ON THE HEART**
BIOREAKTOR-SCREENING-PLATTFORM ZUR MODELLIERUNG DER BIOLOGIE MENSCHLICHER SYSTEME UND ZUM SCREENING AUF INOTROPE WIRKUNGEN VON WIRKSTOFFEN AUF DAS HERZ
PLATEFORME DE CRIBLAGE DE BIOREACTEUR POUR MODELISER LA BIOLOGIE DES SYSTEMES HUMAINS ET POUR CRIBLER DES EFFETS INOTROPES D'AGENTS SUR LE COEUR

(30) Priority: 29.11.2017 US 201762592083 P; 12.01.2018 US 201862616812 P
(43) Date of publication of application: 07.10.2020
(73) Proprietor: Novoheart International Limited, Hong Kong (HK)
(72) Inventor: TRAN, David, D., Aliso Viejo, CA 92656 (US); COSTA, Kevin, D., New York, NY 10026 (US); LI, Ronald, A., Hong Kong (HK)
(74) Representative: Grünecker Patent- und Rechtsanwälte PartG mbB
(86) International application number: PCT/IB2018/001535
(87) International publication number: WO 2019/106438

(56) References cited:
- WO-A1-2017/059171
- WO-A2-2019/186283
- CN-A- 104 888 276
- US-A1- 2010 184 200
- US-A1- 2017 002 330
- US-A1- 2017 260 488
- US-A1- 2017 260 488
- FLORIAN WEINBERGER ET AL: "Engineering Cardiac Muscle Tissue : A Maturating Field of Research", CIRCULATION RESEARCH, vol. 120, no. 9, 28 April 2017 (2017-04-28), US, pages 1487 - 1500, XP055615379, ISSN: 0009-7330, DOI: 10.1161/CIRCRESAHA.117.310738
- AHUJA N ET AL: "Multiview Panoramic Cameras Using Mirror Pyramids", IEEE TRANSACTIONS ON PATTERN ANALYSIS AND MACHINE INTELLIGENCE, IEEE COMPUTER SOCIETY, USA, vol. 26, no. 7, 1 July 2004 (2004-07-01), pages 941 - 946, XP011113426, ISSN: 0162-8828, DOI: 10.1109/TPAMI.2004.33
- WEINBERGER, F. ET AL.: "Engineering Cardiac Muscle Tissue:A Maturating Field of Research", CIRCULATION RESEARCH, vol. 120, no. 9, 28 April 2017 (2017-04-28), pages 1487 - 1500, XP055615379
- BOUDOU, T. ET AL.: "A Microfabricated Platform to Measure and Manipulate the Mechanics of Engineered Cardiac Microtissues", TISSUE ENGINEERING: PART A, vol. 18, no. 9; 10, 29 December 2011 (2011-12-29), pages 910 - 919, XP055149621
- BOUDOU, T. ET AL.: "A Microfabricated Platform to Measure and Manipulate the Mechanics of Engineered Cardiac Microtissues", TISSUE ENGINEERING: PART A, vol. 18, 29 December 2011 (2011-12-29), pages 910 - 919, XP055149621
- VANDENBURGH, H. ET AL.: "DRUG-SCREENING PLATFORM BASED ON THE CONTRACTILITY OF TISSUE-ENGINEERED MUSCLE", MUSCLE NERVE, vol. 37, 30 January 2008 (2008-01-30), pages 438 - 447, XP055254042

## Description

### FIELD

The disclosure relates generally to the fields of medical health and cardiac physiology and, more specifically, to the fields of medical devices in providing versatile bioreactor platforms monitoring function of human tissue-engineered organoids and to the field of medical therapy in providing methods of and screening for bioactive compounds, such as compounds or agents having inotropic effects on the heart. The present invention relates to a tissue monitoring system, a method for assaying a compound for bioactivity, and a system for screening a compound for inotropism.

### BACKGROUND

Traditional discovery and development of novel drugs and therapeutics for heart diseases continue to be an inefficient and expensive process. Due to the lack of appropriate human models, cardiotoxicity has been a common leading cause for withdrawal, even for non-cardiovascular (*e.g.,* cancer) drugs. Although such traditional animal models as rodents, dogs and pigs are accessible, major species differences in both anatomy and function exist. Human pluripotent stem cells (hPSC) have been proposed to fill this gap, but conventional two-dimensional cultures and experiments with single cells or disorganized clusters inadequately recapitulate the human cardiac phenotype. As such, previous hPSC-based drug screening models focus on such modalities as single-cell viability and electrophysiological effects, making them useful tools for cardiotoxicity screening. Newer two-dimensional models have also focused heavily on electrophysiology and arrhythmogenic effects for evaluation of cardiotoxicity. To date, only a few hPSC-based drug screening systems have been developed to investigate the effect of drugs on cardiac contractility, though surrogate indices of contractility have been developed for quantification of contractile force at the single-cell level. These hPSC-based drug screening systems include 2D strain-gauge-embedded muscular thin films, cardiac microtissues, cardiac microwires or biowires, miniaturized cardiac muscles (µHM), and force-generating engineered heart tissues (EHT), albeit with sub-physiological functionality when compared to native hear tissues, which is a limitation common to all engineered tissue systems developed to date.

The process of developing and gaining approval to market a therapeutic useful in treating a disease or disorder in humans or other animals is a long, expensive and uncertain process. Despite the delays in bringing new therapeutics to the clinical or veterinary setting for treatment of suffering subjects, the public recognizes the importance of being certain that a new therapeutic will be efficacious with minimum negative side effects. Preclinical pharmaceutical testing currently relies on experimental animals to determine the safety and efficacy of new pharmaceutical drugs. Animal drug testing is a slow, expensive, and unreliable predictor of safe consumption of drugs by human patients. This is apparent upon recognition that adverse effects are the main cause of drug failure in clinical trials of candidate therapeutics for treating diseases and disorders. Thus, there is an urgent need for developing supplemental methods for preclinical pharmaceutical testing in evaluating the safety and efficacy of experimental therapeutics.

Human pluripotent stem cell (hPSC) technology provides exciting new opportunities for *in vitro* therapeutic screening platforms. These cells offer many advantages over animal models, including human origin, culture adaptation, and the ability to create patient-specific lines for inherited disorders (*e.g.,* long QT syndrome). Differentiated cells (*e.g.,* heart, brain, nerve, liver, kidney, adrenal gland, stomach, pancreas, gall bladder, lung, small intestine, colon, bladder, prostate, uterus, blood, vascular, tumor, eye, or skin) sourced from hPSC have been used with tissue-engineering methods to recapitulate aspects of the three-dimensional environment of native tissues in order to better mimic human function. A major bottleneck in the adoption of these new predictive platforms for therapeutic screening is the lack of tools capable of culturing and monitoring the function of such tissue models.

To further enhance the predictive capabilities of *in vitro* therapeutic screening, tools to support a modular systems-biology approach to predict multi-organ or full "body" response are desired. A drawback of current *in vitro* screening platforms is that they typically evaluate the response of a single organ. In contrast, the body is an intricate and dynamic system of multiple organs with complex interactions. For example, certain drugs are more active as metabolite derivatives (*e.g.,* doxorubicin compared to doxorubicinol) and a simple screening platform may not properly replicate the clinical pharmacological pathway. Some work has begun connecting different organ-testing platforms (*e.g.,* organ-on-a-chip) but have only done so at the microscale level (*e.g.,* microfluidic device), unsuitable for a systems biology approach of larger tissue-engineered organoids.

Therefore, there remains an urgent need to develop new therapeutic testing platforms that enable better prediction of the effects of candidate therapeutics on *in vitro* tissue-engineered organoids and new tools to permit "organ" to "organ" interaction for analysis of holistic functional response (*i.e.,* whole body response). Further, there remains a need for accurate and efficient screens for compounds or agents having desired effects on organs effectively modeled using *in vitro* tissue-engineered organoids or tissues.

Although much effort is expended to advance cardiac care, a need continues to exist in the art for more rapid and more reliable identification of cardiac drugs with inotropic effects. Positive inotropes are agents that strengthen the contractile force of the heart, and are commonly prescribed for patients suffering from congestive heart failure or cardiomyopathies, or in some cases, to patients who have had a recent heart attack. Negative inotropes, which weaken heart contractions, are used to treat hypertension and angina. These inotropic effects could be efficacious if applied to the appropriate conditions, but could also be toxic to other patients. The ability to sensitively detect these effects is therefore invaluable for any cardiac efficacy/toxicity screen.

A cardiac organoid containing 3-D matter of adult human heart tissue is known from US 2017/002330 A1.

### SUMMARY

The invention is set out in the appended claims. Any "embodiment" or "example" which is disclosed in the description but not covered by the claims should be considered as presented for illustrative purpose only. The present disclosure provides a three-tiered system, and associated methods, to facilitate drug discovery/screening using engineered human ventricular cardiac tissue strips (hvCTSs) in a first tier screen useful for rapid determinations of compounds having an inotropic effect and amenable to large-throughput screening formats. This first-tier screen is typically combined with a second-tier screen of the compound using a human ventricular cardiac organoid chamber (hvCOC) as disclosed herein. The two-tiered screen for compounds having inotropic effects permits a staged focusing on compounds of interest while obtaining data on the effects of a compound on cells, *e.g.,* cardiomyocytes such as human ventricular cardiomyocytes, found in the first-tier screen using an hvCTS, and combining that data with the effects of the compound on the higher order biological structure of a related organoid, such as a cardiac organoid, to confirm the findings of the first-tier screen and to reveal any organoid- or organ-level effects not apparent from the cells used in the first-tier screen. This approach not only allows for the rapid, progressive focus on compounds having inotropic effects, it assesses the effects of the compounds in two different, yet relevant, contexts, *i.e.,* the cell-based context and the organoid- or organ-based context. Using this approach, the results are more reliable in being both more accurate and more reproducible than results obtained using any single-stage screen, such as the compound screening systems and methods currently in use. The disclosure further provides for the possibility of a third-tier screen optimally combined with the first- and second-tier screens. In the third-tier screen, the hvCOC organoid screen is expanded to multiple organoids using a multi-organoid hvCOC system. The third tier screen yields data that is even more reliable than the data obtained from two-tiered screening. In addition, the multi-organoid hvCOC format allows for multiple organoids of the same type, *e.g.,* cardiac organoids, to be used in the screen and/or for different organoids to be used in the third-tier screen at the same time, using the versatile multi-organoid hvCOC system disclosed herein. (As used herein, "organoid" typically refers to an organ-like biomaterial, but the term can also refer to a tissue, which can be considered an organ-like biomaterial. The meaning of the term used herein will be apparent from the context of its usage.)The technology is versatile in being suited for the identification of compounds having positive inotropic effect and compounds having negative inotropic effect. In some embodiments, the human ventricular cardiac tissues comprise hPSC-derived ventricular cardiomyocytes (VCMs). The single-cell properties of such cells, such as electrophysiology (action potential, Ca²⁺ handling), transcriptome, proteome, and the like, have been extensively characterized. As disclosed herein, various cells, *e.g.*, human ventricular cardiomyocytes, may be used in developing the organoids used in the second-tier hvCOC system and in developing the organoids used in the third-tier multi-organoid hvCOC system.

The hvCOC system used in the second and third tiers of the multi-tiered systems and methods of the disclosure is the first platform that simultaneously characterizes multiple *in vitro* tissue-engineered tissues or organoids, including a mirror arrangement together with a single detection device. Equipped with a fluidic exchange network, organoids are interconnected in this platform to model a "mini-human" system that simulates systemic drug responses in human patients that is useful in replacing animal testing as the default *in vivo* model. The semi-automated platform includes multiple features to aid in investigating functional response to delivered drugs, such as environmental control (*e.g.,* temperature and CO₂), high-speed camera, synchronized pressure-volume recordings, interconnected fluidic exchange system, drug perfusion, intra-organoid pressure control, mechanical stimulation, and electrical stimulation. These features are designed to improve culture handling, permit examination of long-term drug exposure of tissues or organoids, and allow simultaneous multi-tissue and/or multi-organ drug response. Current *in vitro* therapeutic screening typically assesses only the acute response of single tissues or organoids, which makes scaling up challenging and costly. By using a single camera with a mirror arrangement for multi-organoid imaging, this system is more scalable than currently available designs.

To develop next-generation *in vitro* human models, the bioreactor platform includes a modular organoid cartridge system and a fluid exchange network that enables a flexible systems biology approach. "Plug-and-play" organoid cartridges expedite the process of imaging various tissue and/or organoid combinations of interest within the bioreactor. In addition, the circulation and exchange of media between tissues or organoids recapitulates the human circulatory system. Signaling factors and metabolites can be freely exchanged between tissues or organoids and can affect the drug response of one or more tissues or organoids. For example, the metabolite doxorubicinol is known to be more cardiotoxic than the chemotherapeutic doxorubicin itself. Such "body-in-a-jar" technology facilitates drug discovery and precision, or personalized, medicine efforts, and is superior to organ-on-a-chip technologies that often fail to fully recapitulate organ function owing to the lack of three-dimensional organization.

New molecular entities are characterized using clinically relevant endpoints (*e.g.,* ejection fraction in heart tissue, permeability in lung tissue) and then classified using automated computer algorithms (*e.g.,* machine learning) trained to detect patterns of bioactivity and toxicity. The multi-organoid imaging platform disclosed herein increases throughput and is useful for higher content screening. By improving throughput, the system becomes more accessible to preclinical pharmaceutical screening. The platform is also used to probe basic biology in tissue-engineered human constructs.

In addition to the tier one hvCTS system and method, the disclosure provides a versatile bioreactor platform for developing engineered organoid tissues that more closely mimic the *in vivo* structure and function of the corresponding human organ. The bioreactor platform allows for control of a variety of environmental variables and permits varied probes and monitors for use in real-time or end-point monitoring of tissue or organoid function. Additionally, the disclosure provides for improved environmental control in providing for the control of temperature and the control of various gases, *e.g.,* CO₂ and oxygen. A well-controlled environment permits stable culturing of cells and therefore long-term acquisition for drug screening is enabled. With a combination of a high-speed camera and pressure transducers, the disclosure provides a method to combine spatiotemporal movement of shifting tissues or organoids (*e.g.,* contracting heart organoid) with pressure recordings to measure pressure-volume relationships. In addition, the disclosure provides a sophisticated system for fluidic exchange within the bioreactor platform through the coordinated use of fluidic pumps, three-way valves and fluid tanks, as opposed to some systems using simple hydrostatic pressure systems. The fluidic exchange system also provides for connection of any number of organoids within the bioreactor. The fluidic exchange system of the disclosure provides the additional functions of controlling media delivery for feeding, aspirating media, the mixing of bioactive components (*i.e.,* therapeutics), and the injection of bioactives on an acute schedule (*e.g.,* a bolus) or a chronic schedule (*e.g.,* perfusion). Bioactive components may include, but are not limited to, drug compounds, viral vectors, conditioned media, progenitor cells, and extracellular vesicles. The fluidic exchange system also provides for cleaning, rinsing or washout of fluidic lines. In addition, the disclosure provides for mechanical stimulation of developing tissues or organoids by applying a method for mechanical stretching to tissues or organoids with cavities. By applying mechanical stretching, the disclosure provides a means for manipulating tissues or organoids, given that mechanical stretch can act as a mechanotransduction signal. In contrast to electrical pacing of human cardiac organoids via field stimulation, the disclosure provides for point stimulation of such organoids, resulting in a more precise and refined stimulation of organoid tissues. With point stimulation, electrical conduction measurements within tissues or organoids (*e.g.,* brain, heart) can be accomplished, for example by using optical mapping techniques. Further, the application of machine learning principles in the analysis of tissue or organoid behavior according to the disclosure is expected to improve evaluation of therapeutic response outcomes by comprehensively analyzing and understanding high-dimensional parameter spaces. All of the benefits are provided in an integrated package by the disclosure, representing a significant advance in the field of therapeutic screening, including new methods, *i.e.,* experimental assays, that are expected to lead to improved prevention, treatment and/or amelioration of symptoms of various diseases and conditions.

In one aspect, the disclosure provides a system for screening a compound for inotropism comprising: (a) a first-stage screening apparatus comprising: (i) a biocompatible gel comprising a plurality of cardiomyocytes; (ii) a biocompatible support apparatus for suspending the biocompatible gel, wherein the biocompatible gel and biocompatible support apparatus form a cardiac tissue strip; (iii) a detection device for detecting movement of the biocompatible gel; and (iv) an electrical power source for applying an electrical pacing stimulus to the biocompatible gel; and (b) a second-stage screening apparatus comprising: (v) at least one organoid module comprising at least one organoid cartridge, wherein the organoid cartridge comprises a media inlet, a media outlet, and at least one wall compatible with an external detection device, wherein each organoid cartridge comprises a biological material comprising at least one human cell that is a human embryonic stem cell, a human adult stem cell, a human induced pluripotent stem cell, a cell derived from a human tissue, or a progenitor cell of a human tissue, and wherein at least one organoid cartridge comprises cardiac biological material; (vi) a mirror arrangement for simultaneous monitoring of any biological development of the biological material in each organoid cartridge; and (vii) a detection device for observing the monitored biological development of the biological material in each organoid cartridge. In some embodiments, the cardiomyocytes are human cardiomyocytes, such as human ventricular cardiomyocytes. In some embodiments, the human cardiomyocytes are derived from at least one human pluripotent stem cell. In some embodiments, the cardiomyocytes are present at a concentration of at least 10⁶ cells/ml. In some embodiments, the biocompatible gel comprises matrigel, such as biocompatible gels wherein the matrigel is present at a concentration of at least 0.5 mg/ml or 1 mg/ml. The matrigel can be obtained from stock solutions containing, *e.g.,* at least 5 mg/ml or at least 10 mg/ml matrigel. In some embodiments, the biocompatible gel further comprises collagen. The disclosure also provides embodiments wherein the collagen is type I human collagen. Some embodiments comprise biocompatible gels wherein the collagen is present at a concentration of at least 1 mg/ml, such as a concentration of about 2 mg/ml. In some embodiments, the same biocompatible gel composition is used in forming both the cardiac tissue strip in the first stage of screening and the cardiac organoid in the second stage of screening. In some embodiments, the support apparatus is at least two vertical support members (e.g., embodiments in which there are two vertical support members), and those vertical support members may be made of polydimethylsiloxane. In some embodiments, the vertical support members (*e.g.,* two vertical support members) are approximately circular in cross-section with a diameter of about 0.5 mm. In some embodiments, the cardiac tissue strip is about 26.5 mm in length by about 16 mm in width by about 6 mm in height, such as a cardiac tissue strip that is 26.5 mm in length by 16 mm in width by 6 mm in height. In some embodiments, the detection device is a high-speed camera.

In some embodiments of the system, the mirror arrangement of the second-stage screening apparatus comprises at least one pyramidal mirror. The disclosure also contemplates embodiments wherein the biological material is at least one cardiac tissue or at least one cardiac organoid. In some embodiments, the heart organoid is cultured in the same system with other organoids. Thus, some embodiments further comprise a second organoid that is a heart, a brain, a nerve, a liver, a kidney, an adrenal gland, a stomach, a pancreas, a gall bladder, a lung, a small intestine, a colon, a bladder, a prostate, a uterus, a tumor, an eye, skin, blood, or a vascular organoid, such as wherein the second organoid is a heart organoid. In some embodiments, the second-stage screening apparatus further comprises an electrode in adjustable relation to the tissue or organoid in at least one organoid cartridge. In some embodiments, the second-stage screening apparatus further comprises a temperature control element, a light source, a module access port, or any combination thereof. In some embodiments, the system further comprises a data processor in electronic communication with the detection device, a temperature control element, a light source, a module access port or any combination thereof. Also comprehended are embodiments wherein the detection device is a digital camera, at least one pressure transducer, or a combination of a digital camera and at least one pressure transducer. In some embodiments, the system further comprises a tissue comprising at least one human cell. In some embodiments, the system further comprises a monitor. Embodiments of the system also comprise a plurality of organoid modules. In some embodiments, the system further comprises an interconnected fluid exchange network, wherein the network comprises a plurality of fluid lines, a plurality of valves, at least one pump, and at least one fluid tank. Embodiments are also contemplated wherein the system further comprises a port for introduction of a compound. In some embodiments, the interconnected fluid exchange network comprises fluid communication between at least two organoid cartridges. In some embodiments, the fluid is media. In some embodiments, the fluid exchange network provides automated media exchange. Some embodiments of the system further comprise a gas pressure controller. In some embodiments, the gas pressure controller controls the concentration of at least one of O₂ and CO₂ in at least one module or in one or more organoid cartridges. In some embodiments, the system further comprises a drug perfusion apparatus for delivery of a compound to the cell, tissue, or organoid.

Another aspect of the disclosure is drawn to a method of making a cardiac tissue strip comprising: (a) providing a biocompatible mold approximately 26.5 mm in length by approximately 16 mm in width by approximately 6 mm in height; (b) forming a biocompatible gel conforming to the mold, wherein the biocompatible gel comprises matrigel, collagen and a plurality of cardiomyocytes; and (c) affixing at least two vertical support members to the biocompatible gel, thereby forming a cardiac tissue strip. In some embodiments of the method, the vertical support members are affixed to the biocompatible gel by embedding the vertical support members in the biocompatible gel formulation prior to gelation. In some embodiments, the vertical support members are affixed to the biocompatible gel by adhesion or by mechanical attachment.

Yet another aspect of the disclosure is drawn to a method of screening for a compound having inotropic effect comprising: (a) administering the test compound at a specific concentration (or an equivalent volume of vehicle) to a cardiac tissue strip; (b) measuring spontaneous contraction of the cardiac tissue strip in the absence of electrical pacing; (c) pacing the cardiac tissue strip as disclosed herein with an electrical stimulus at a pacing frequency of 0.5 Hz, 1.0 Hz, 1.5 Hz or 2.0 Hz, and measuring contraction of the cardiac tissue strip; (d) repeating the above steps at increasing concentrations of the test compound; (e) eliminating data acquired from cardiac tissue strips with low contractile force, for example 0.01 mN when in the absence of treatment; (f) plotting dose-response curves (contractile force against dose), and comparing between compound- and vehicle-treated dose-response curves, to determine the presence or absence of inotropic activity (by differentiating bona fide pharmacological effects of the test compound from artefactual effects of vehicle treatment); and (g) administering the potential inotropic compound to the tissue or organoid in the second-stage screening apparatus disclosed herein and monitoring the response of the heart tissue or organoid to the compound, wherein modified contractility (for example developed pressure, stroke volume, and stroke work in a cardiac organoid) identifies the potential inotropic compound as an inotropic compound. The disclosure also provides a method of screening for a compound having inotropic effect comprising: (a) pacing a cardiac tissue strip according to claim 1 with an electrical stimulus at a pacing frequency of 0.5 Hz, 1.0 Hz, 1.5 Hz or 2.0 Hz in the presence or absence of a candidate inotropic compound; (b) detecting any movement of the paced cardiac tissue strip in the presence or absence of the candidate inotropic compound; (c) comparing the movement of the paced cardiac tissue strip in the presence of the candidate inotropic compound to the movement of the paced cardiac tissue strip in the absence of the candidate inotropic compound; (d) determining that the candidate inotropic compound is a potential inotropic compound when the movement of the paced cardiac tissue strip differs in the presence of the compound compared to the movement of the paced cardiac tissue strip in the absence of the compound; and (e) administering the potential inotropic compound to the tissue or organoid in the second-stage screening apparatus as disclosed herein and monitoring the response of the tissue or organoid to the compound, wherein modified contractility identifies the potential inotropic compound as an inotropic compound.

The following disclosed embodiments describe embodiments of each of the above screening methods. In some embodiments, the pacing frequency is 1.0 Hz. In some embodiments, the inotropic compound is identified as a potential negative inotropic compound when the movement of the paced cardiac tissue strip is less in the presence of the inotropic compound than in the absence of the inotropic compound. In some embodiments, the inotropic compound is identified as a negative inotropic compound when (a) the movement of the paced cardiac tissue strip is less in the presence of the inotropic compound than in the absence of the inotropic compound; and (b) the tissue or organoid in the second-stage screening apparatus exhibits reduced contractility in the presence of the inotropic compound compared to the absence of the inotropic compound. In some embodiments, the inotropic compound is identified as a potential positive inotropic compound when the movement of the paced cardiac tissue strip is greater in the presence of the inotropic compound than in the absence of the inotropic compound. In some embodiments, the inotropic compound is identified as a positive inotropic compound when (a) the movement of the paced cardiac tissue strip is more in the presence of the inotropic compound than in the absence of the inotropic compound; and (b) the tissue or organoid in the second-stage screening apparatus exhibits increased contractility in the presence of the inotropic compound compared to the absence of the inotropic compound. In some embodiments of this aspect of the disclosure, the compound is a drug, a viral vector, conditioned media, extracellular vesicles, additional cells, or any combination thereof. In some embodiments, the heart tissue or organoid is connected to (*i.e.,* in fluid communication with) other organoids, such as liver, to detect the systems-level response to the test compound.

In other aspects, the disclosure provides a system and method involving the hvCOC of the second- or third-tier of the multi-tier system and method described herein. One of these aspects is drawn to a tissue monitoring system comprising (a) at least one organoid module comprising a plurality of organoid cartridges, wherein each organoid cartridge comprises a fluid (*e.g.,* media) inlet, a fluid (*e.g.,* media) outlet, and at least one wall compatible with an external detection device, wherein a plurality of the organoid cartridges each comprise a biological material comprising at least one human cell, wherein the cell is a human embryonic stem cell, a human adult stem cell, a human induced pluripotent stem cell, a cell derived from a human tissue, or a progenitor cell of a human tissue; (b) a mirror arrangement for simultaneous monitoring of any biological development of a biological material in each of at least two organoid cartridges; and (c) a detection device for observing the monitored biological development of the biological material in each of at least two organoid cartridges. A wall compatible with an external detection device is a generally flat, generally vertical, physical barrier at the perimeter of an organoid cartridge that effectively prevents fluid (*e.g.,* media) loss through leakage from an organoid cartridge while being effectively permissive to any wavelength of electromagnetic radiation detectable by the external detection device, such as a camera (*e.g.,* a digital camera). Exemplary walls compatible with an external detection device are glass or any effectively transparent thermosetting or thermoplastic plastic. The biological material is any cell or group of like or unlike cells, tissues, organoids, organ systems, and is typically human in origin. Biological development of the biological material means any growth in number or size of cells or multi-cellular structures, differentiation or change in structure (*e.g.,* size, shape, color, topology) or function/behavior (*e.g.,* rhythmic or arrhythmic contraction) of the biological material relative to its state at a prior point in time. The tissue monitoring system disclosed herein provides a system comprising a higher order plurality of biological cells such as would be found in a tissue or organ. Thus, the tissue monitoring system includes systems for monitoring a tissue, an organoid, or an organ. In some embodiments, the system comprises a tissue-engineered organoid, *e.g.,* a three-dimensional tissue-engineered organoid, or a tissue, *e.g.,* an *ex vivo* tissue. An organoid comprises a cell(s) and a tissue(s) characteristic of an organ such that the organoid exhibits at least one biological function of the corresponding organ. The mirror arrangement can be any arrangement of a mirror or mirrors that provides for the monitoring of biological material in the form of one or more cells, tissues, organoids or organs in a plurality of organoid cartridges using fewer detection devices than organoid cartridges to improve the efficiency and lower the cost of monitoring. The system described in this paragraph for use in tissue monitoring can also be used as the hvCOC system of the second tier, and of the third tier, of the multi-tiered system described herein as useful in screening compounds for inotropic activity.

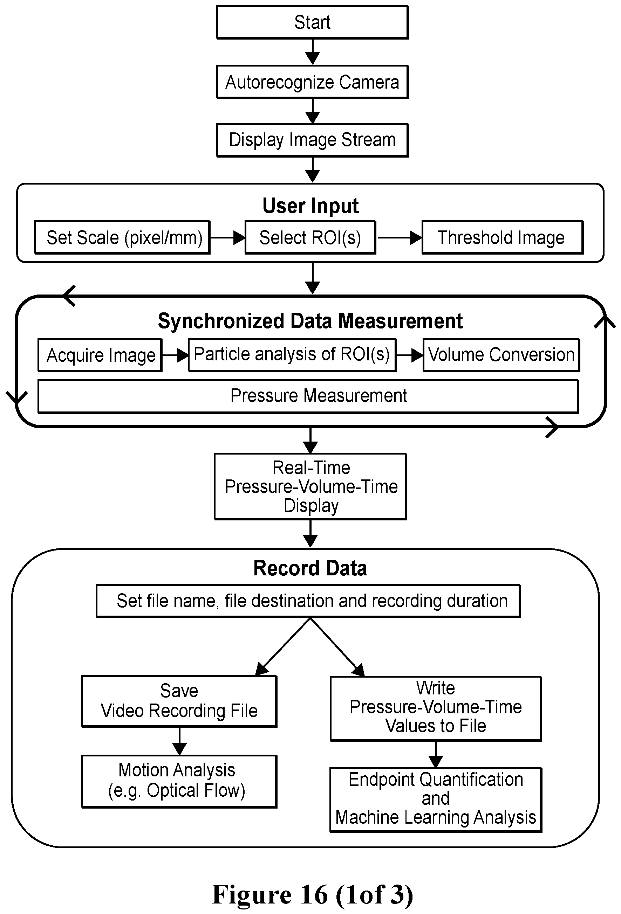

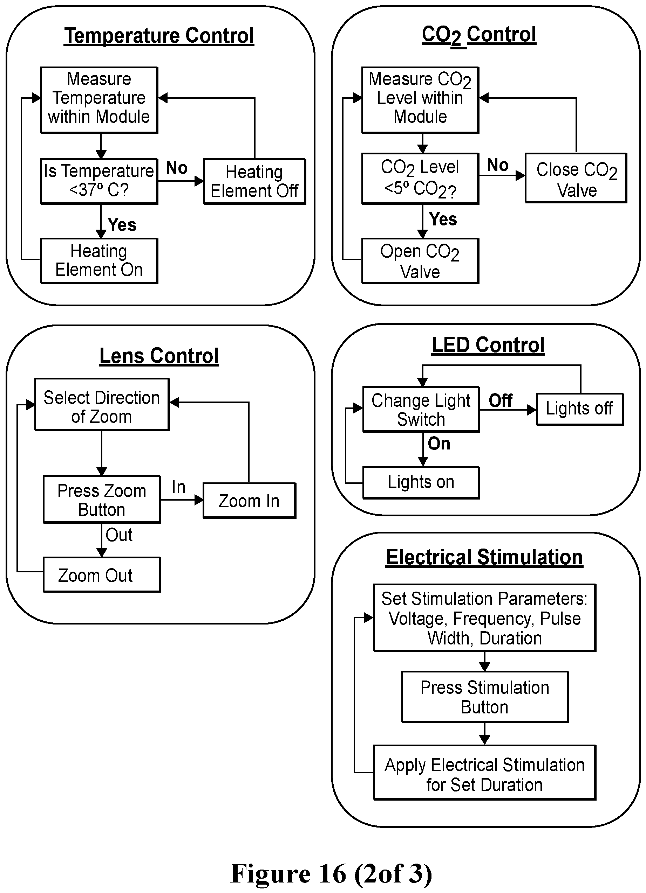

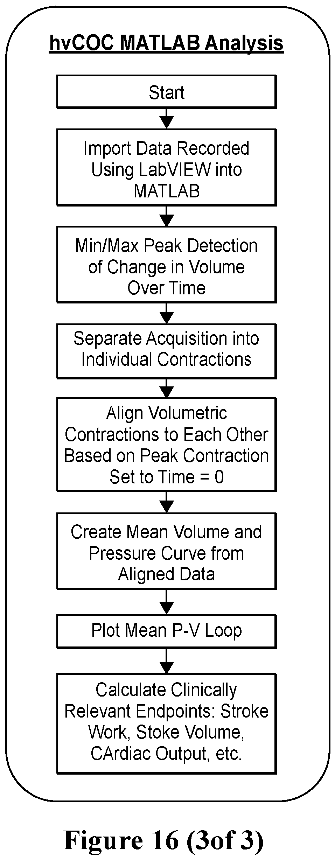

In some embodiments, the mirror arrangement comprises at least one pyramidal mirror. In some embodiments, the biological material is at least one tissue or at least one organoid, such as embodiments wherein the organoid is a heart, a brain, a nerve, a liver, a kidney, an adrenal gland, a stomach, a pancreas, a gall bladder, a lung, a small intestine, a colon, a bladder, a prostate, a uterus, a tumor, an eye, skin, blood, or a vascular organoid. In some embodiments, the organoid is a heart organoid. In some embodiments, the system further comprises an electrode in adjustable relation to the cell, tissue, or organoid in at least one organoid cartridge, *e.g.,* for point stimulation (*e.g.,* point electrical stimulation) and/or point monitoring of the behavior of at least one cell of the tissue or organoid. In some embodiments, the detection device is a recording device, such as a camera. In some embodiments, the recording device is a digital camera, at least one pressure transducer, or a combination of a digital camera and at least one pressure transducer. In typical embodiments wherein the recording device is at least one pressure transducer, there is a 1:1 correspondence between the pressure transducers and the organoid cartridges, *i.e.,* one pressure transducer per organoid cartridge comprising a cell, tissue or organoid being monitored. In some embodiments, the system further comprises a temperature control element (*e.g.,* a heater), a light source (*e.g.,* LED lamps or lights), a module access port, or any combination thereof. In some embodiments, the system further comprises a data processor in electronic communication with the detection device, a temperature control element, a light source, a module access port or any combination thereof. The electronic processor provides software- and/or hardware-based control of the elements of the system and devices to control the environment, such as by controlling fluid (*e.g.,* media) flow by controlling pumps and valves in an interconnected fluid exchange network, and/or controlling a heater, lights, a detection device such as a camera, as well as by processing results to yield a form useful for analysis by operators (Figure 16).

The hvCOC system disclosed herein may further comprise a tissue comprising at least one human cell. In some embodiments, the system further comprises a monitor. In some embodiments, the system comprises a plurality of organoid modules. In some embodiments, the system further comprises a media mixer, such as a magnetic stirring apparatus or a turntable.

In some embodiments, the hvCOC system disclosed herein further comprises an interconnected fluid exchange network, wherein the network comprises a plurality of fluid lines, a plurality of valves, at least one pump, and at least one fluid tank. An interconnected fluid exchange network includes any combination of fluid lines, valves, pumps, tanks and/or organoid cartridges as disclosed herein that are collectively capable of moving fluid through more than a single path, and preferably between or among a plurality of organoid cartridges. Typical embodiments of the hvCOC system comprising the interconnected fluid exchange network control fluid flow using the data processor to control at least one valve and at least one pump. In some embodiments, the hvCOC system further comprises a port for introduction of a compound, such as a therapeutic, candidate therapeutic, drug, or candidate drug. In some embodiments, the interconnected fluid exchange network comprises fluid communication between at least two organoid cartridges, and in typical embodiments, the fluid communication is controlled by the data processor controlling at least one valve and at least one pump. In some embodiments, the interconnected fluid exchange network provides a partially common fluid delivery path for at least two organoid cartridges, a partially common fluid removal path for at least two organoid cartridges, or both a partially common fluid delivery path and a partially common fluid removal path for at least two organoid cartridges. In typical embodiments, the fluid is media. In some embodiments, the fluid exchange network provides automated media exchange, such as by using the data processor to control at least one valve and at least one pump to thereby control fluid, *e.g.,* media, exchange. In some embodiments, the hvCOC system further comprises a gas pressure controller, such as a gas pressure controller that controls the concentration of at least one of O₂ and CO₂ in at least one module or in one or more organoid cartridges. In some embodiments, the hvCOC system further comprises a plurality of module access ports. Embodiments of the hvCOC system disclosed herein that further comprise a drug perfusion apparatus for delivery of a therapeutic to the cell, tissue, or organoid are also contemplated. In typical embodiments, the drug perfusion apparatus comprises the port for introduction of a compound in combination with at least one fluid line extending into an organoid cartridge comprising a tissue, organoid or organ to be exposed to the compound, at least one valve, and at least one pump. Optionally, the drug perfusion apparatus also comprises a fluid line and, optionally, at least one valve and at least one pump, to remove fluid, *e.g.,* media, from the organoid cartridge.

Another aspect of the disclosure is drawn to a method for assaying a compound for bioactivity comprising administering a compound to the cell, tissue, or organoid in the hvCOC system disclosed herein and monitoring the response of the cell, tissue or organoid to the compound. In some embodiments, the compound is a drug, a viral vector, conditioned media, extracellular vesicles, additional cells, or any combination thereof. In several embodiments, the compound is delivered in conjunction with a chemical or biologic that is known to have an effect on the biological material in the organoid cartridge to which the compound is being provided. In such embodiments, the effect of the compound on the change induced by the chemical or biologic is monitored. In these embodiments, the compound and the chemical or biologic may be co-administered or the administrations may be offset in time. In some embodiments, the bioactivity is modulation of a function of the cell, tissue, or organoid, thereby identifying the compound as a therapeutic for treatment of a disorder of the organ cognate of the cell, tissue, or organoid. In some embodiments, the assay measures the toxicity of the compound.

A related aspect of the disclosure is directed to a method for assaying a compound for bioactivity comprising administering a compound to a plurality of cells, tissues, or organoids in the hvCOC system disclosed herein through an interconnecting fluid exchange network and monitoring the response of the cells, tissues, or organoids to the compound. In some embodiments, the compound is a drug, a viral vector, conditioned media, extracellular vesicles, additional cells, or any combination thereof. The compound may be delivered with or without a chemical or biologic that has a known effect on the biological material in the organoid cartridge receiving the compound. In some embodiments, the bioactivity is modulation of a function of the cells, tissues, or organoids, thereby identifying the compound as a therapeutic for treatment of a disorder of the organ cognate of the cells, tissues, or organoids. In some embodiments, the assay measures the toxicity of the compound.

Another aspect of the disclosure is an apparatus for monitoring organoid function comprising at least one organoid module comprising (a) a plurality of organoid cartridges, wherein each organoid cartridge comprises a fluid (*e.g.,* media) inlet, a fluid (*e.g.,* media) outlet, and at least one wall compatible with an external detection device; (b) a mirror arrangement for simultaneous monitoring of at least two organoid cartridges; and (c) a detection device. In some embodiments, the detection device is a recording device. In some embodiments, the hvCOC system further comprises a temperature control element, a light source, a module access port, or any combination thereof. In some embodiments, the hvCOC system further comprises a data processor in electronic communication with the detection device, a temperature control element, a light source, a module access port or any combination thereof.

Yet another aspect of the disclosure is drawn to an organoid produced using the hvCOC system disclosed herein, wherein the organoid comprises a plurality of differentiated cells. In some embodiments, the organoid is a heart, brain, nerve, liver, kidney, adrenal gland, stomach, pancreas, gall bladder, lung, small intestine, colon, bladder, prostate, uterus, blood, tumor, eye, or skin organoid.

Other features and advantages of the disclosed subject matter will be apparent from the following detailed description, including the drawing. It should be understood, however, that the detailed description and the specific examples, while indicating preferred embodiments, are provided for illustration only.

### BRIEF DESCRIPTION OF THE DRAWING

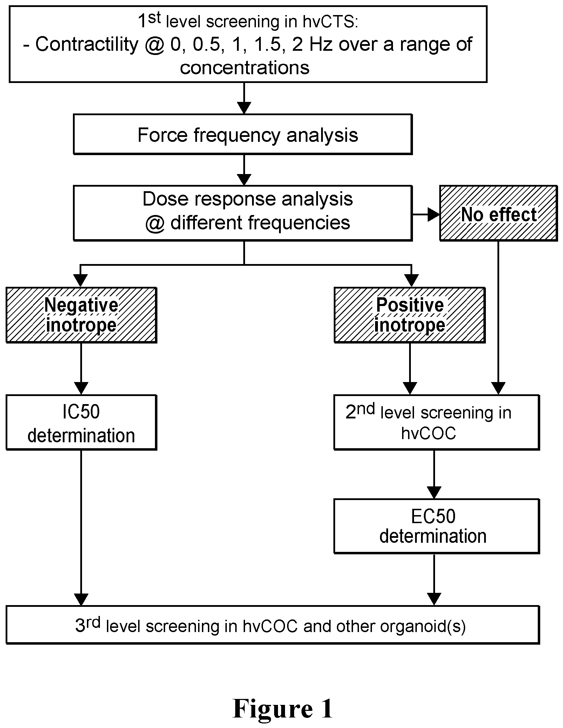

**Figure 1**. Screening platform for inotropic effect of agents on the human heart. A three-tiered flowchart for use of the system comprising the screening platform in methods of screening for negative and/or positive inotropes. The first tier involves hvCTS (human ventricular cardiac tissue strip) screening. Initially, compounds are tested in the hvCTS system using a range of doses and different pacing frequencies (*e.g.,* spontaneous, 0.5, 1, 1.5 and 2 Hz) (n ≥ 5). Next, for compounds determined to be negative inotropes, the IC₅₀ is determined, whereas compounds determined to be positive inotropes or compounds not showing an inotropic effect are subjected to second-tier screening. The second tier screen involves hvCOC (human ventricular cardiac organoid chamber) screening. Initially, compounds are tested using the hvCOC at a range of doses informed by the first-tier screening and at pacing frequencies informed by the first-tier screening (n ≥ 4). Dose-response relationships are analyzed for developed pressure, stroke work and cardiac output. Compounds determined to be positive inotropes in the first-tier screening are confirmed in this second-tier screening using the hvCOC screen. The EC₅₀ is determined for compounds confirmed as positive inotropes. A third-tier screen may also be used. The third-tier screening is an hvCOC screen with a plurality of organoids. In this third-tier screen, compounds are tested using the multi-organoid hvCOC at a range of doses informed by the first-tier screening and at pacing frequencies informed by the first-tier screening (n ≥ 4). Dose-response relationships are analyzed for developed pressure, stroke work and cardiac output. Inotropic effects exhibited by a given compound are thereby confirmed in the presence of other organoids, indicative of the behavior of the compound in one or more organs or organ systems. Consistent with the flowchart, human ventricular cardiac tissue strip (hvCTS) screening is exemplified by the results of testing compounds in the hvCTS system at a range of doses and at different pacing frequencies (*e.g.,* spontaneous, 0.5, 1, 1.5 and 2 Hz) (n≥5). Compounds were determined to be negative inotropes based on hvCTS results and IC₅₀ determinations from fitted dose-response curves. Compounds were confirmed to be positive inotropes based on the hvCTS results and second tier screening using human ventricular cardiac organoid screening, as described in USSN 62/592,083. Compounds showing no inotropic effects in first-tier screening with hvCTS can be confirmed by second tier screening using the human ventricular cardiac organoid screening. Compounds subjected to second tier human cardiac organoid screening were analyzed at a range of dosages and pacing frequencies informed by first tier hvCTS screening disclosed herein. Dose-response relationships were analyzed for developed pressure, stroke work and cardiac output. The results were useful in confirming compounds as positive inotropes, and such compounds were then subjected to EC₅₀ determinations.

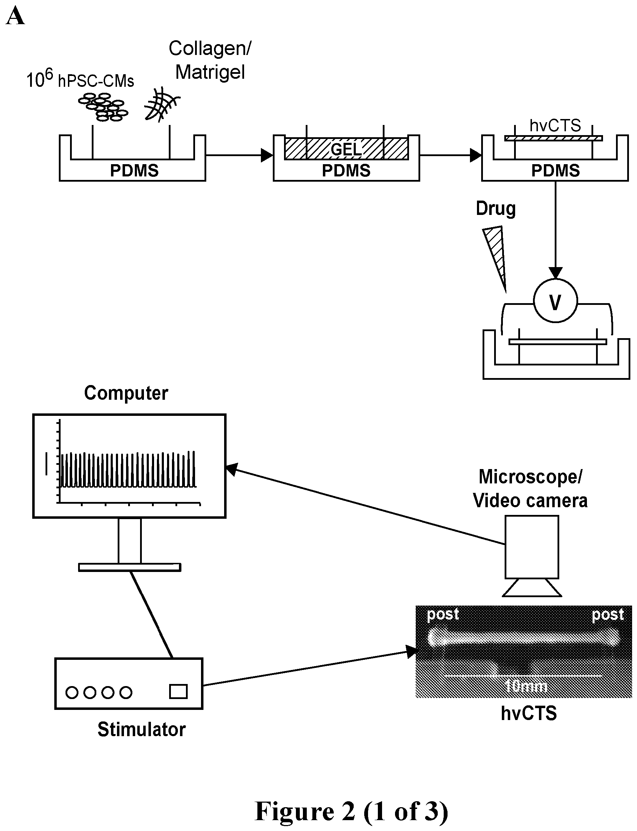

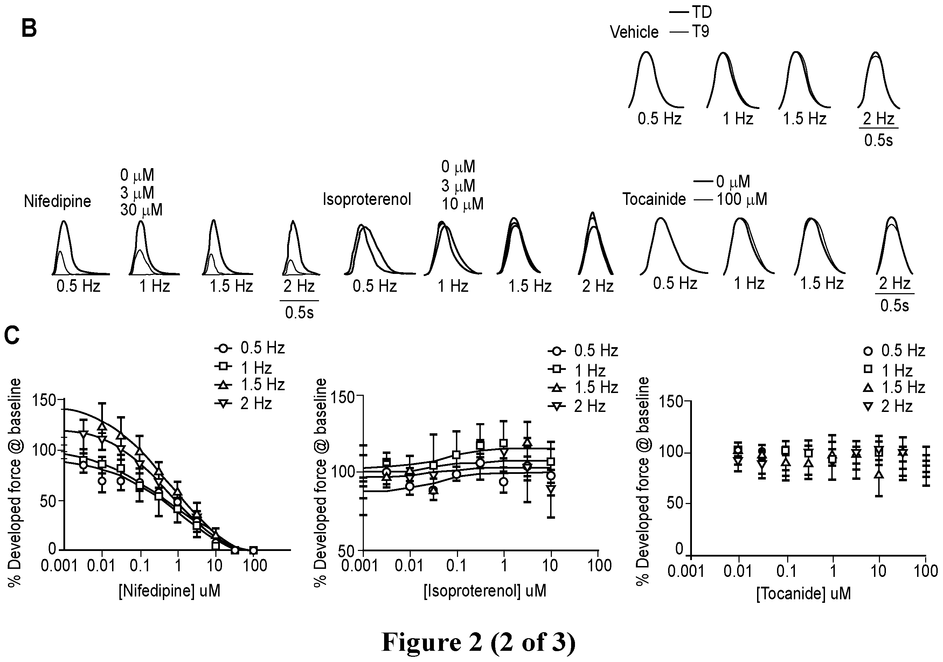

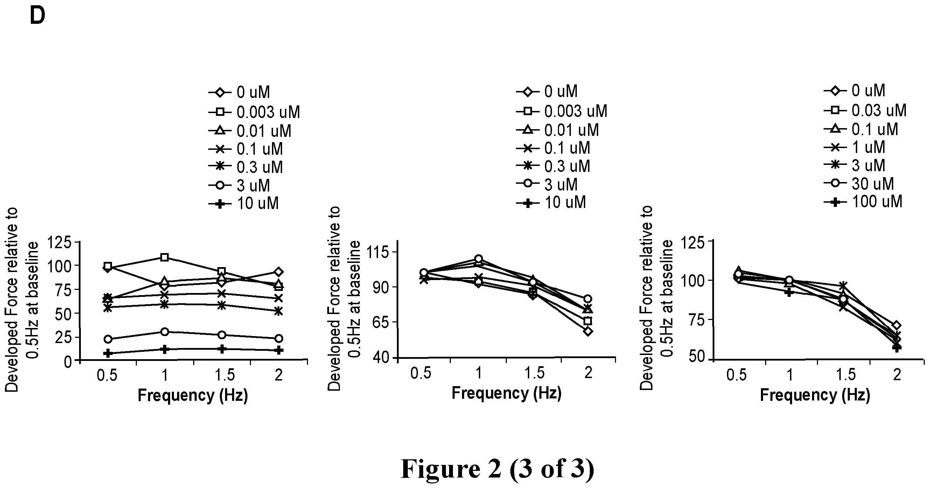

**Figure 2**. Engineering human ventricular cardiac tissue strips (hvCTS) and force measurement. **A**. Engineering human ventricular cardiac tissue strips (hvCTS) and force measurement. Schematic of hvCTS construction in custom-designed PDMS bioreactor mold. One million hPSC-CMs per tissue, mixed with ice-cold bovine collagen type I and Matrigel, polymerized to yield a self-assembled hvCTS held between 0.5-mm diameter flexible end-posts. After 7 days in culture, with the hvCTS maintained in the original molds, test compound screening was conducted wherein twitch force was measured by real-time tracking of end-post deflection as the contracting tissue was subjected to electrical field stimulation and incremental doses of test compound. Force measurements were obtained with the hvCTS maintained in the original molds and force calculated by applying a beam-bending equation from elasticity theory (*F =* {(3π*ER*⁴)/[2*a*²(3*L-a*)]}*δ,* where *F* is the tissue contraction force; *E,* R and *L* represent the Young's modulus, radius and length of the PDMS posts, respectively; *a* is the height of the tissue on the post; *δ* is the measured tip deflection). **B**. Representative tracing showing the contraction profile of nifedipine, isoproterenol and tocainide at 0 Hz to 2 Hz electrical pacing. **C**. Dose-response curves for each drug were fitted for different pacing frequencies. The negative inotrope nifedipine showed a decreasing EC₅₀ with increasing pacing frequency from 2.27 µM at 0.5 Hz to 0.544 µM at 2 Hz. The positive inotrope isoproterenol showed a consistent EC₅₀ with increasing pacing frequency across the range of 0.031 µM to 0.059 µM. Tocainide yielded no dose-dependent effect on contractile force at all tested frequencies. **D.** Force frequency analysis showed nifedipine elicited a decrease in contractile force with increasing concentrations at all pacing frequencies. Isoproterenol elicited an increase in contractile force with increasing concentrations at all tested pacing frequencies. No correlative dose dependent effect was observed with tocainide.

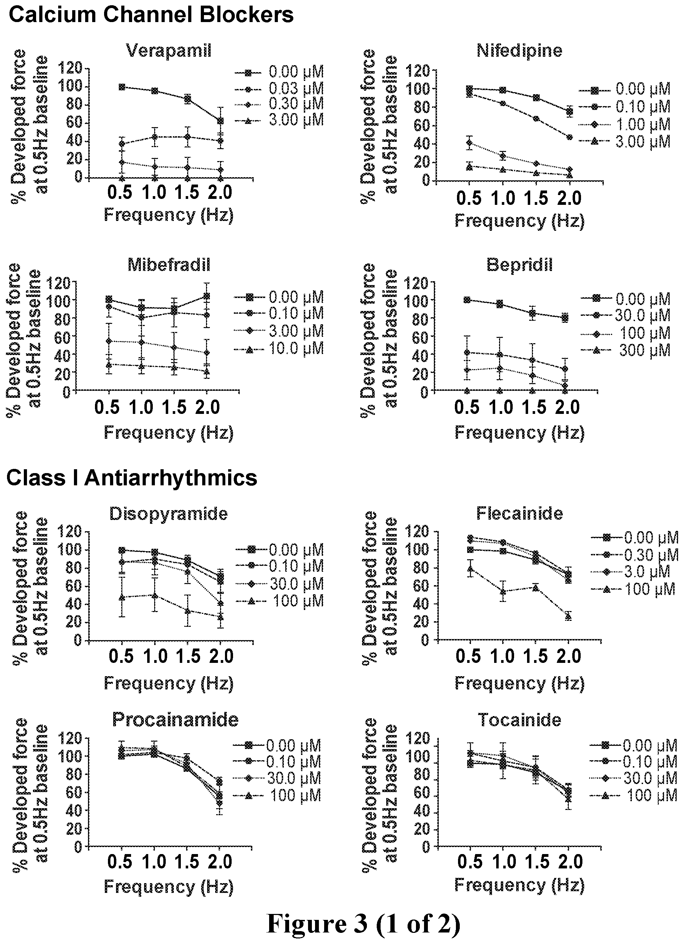

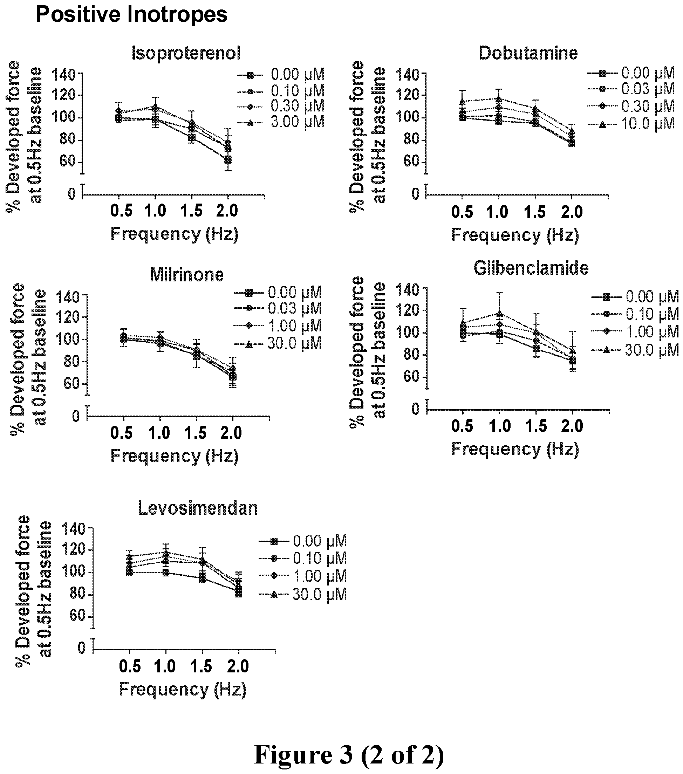

**Figure 3****.** Force-frequency analysis showing known inotropes eliciting changes in contractile force with increasing dosages within the effective concentration range at all pacing frequencies, normalized to the generated force at 0.5 Hz (30 bpm) in drug-free baseline condition. Negative inotropes including calcium channel blockers and 2 out of 4 Class I antiarrhythmics showed a decrease in contractile force with increasing concentrations of the compound at all 4 frequencies. Positive inotropes showed an increase in contractile force with increasing concentrations of the drug at all 4 frequencies. Data show mean±SEM of n=4-8.

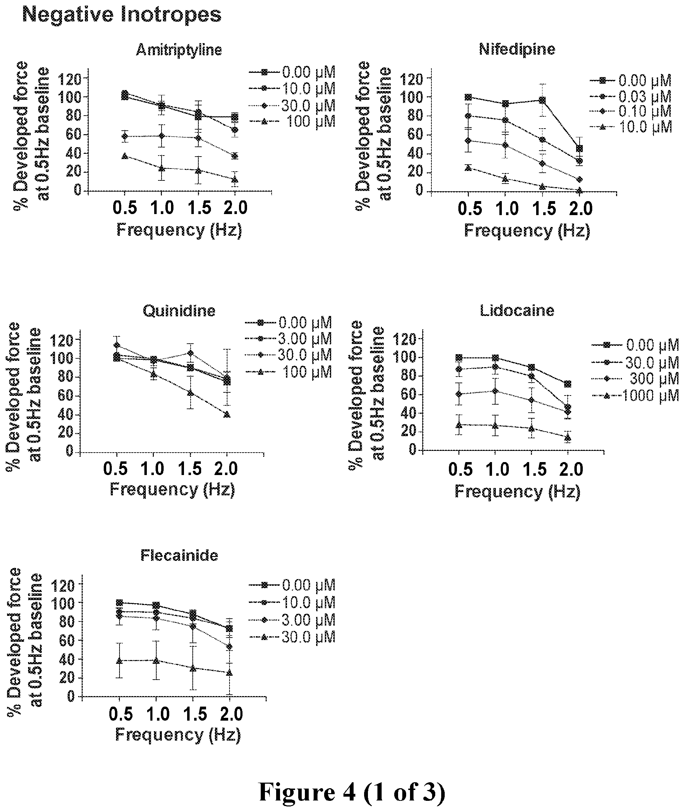

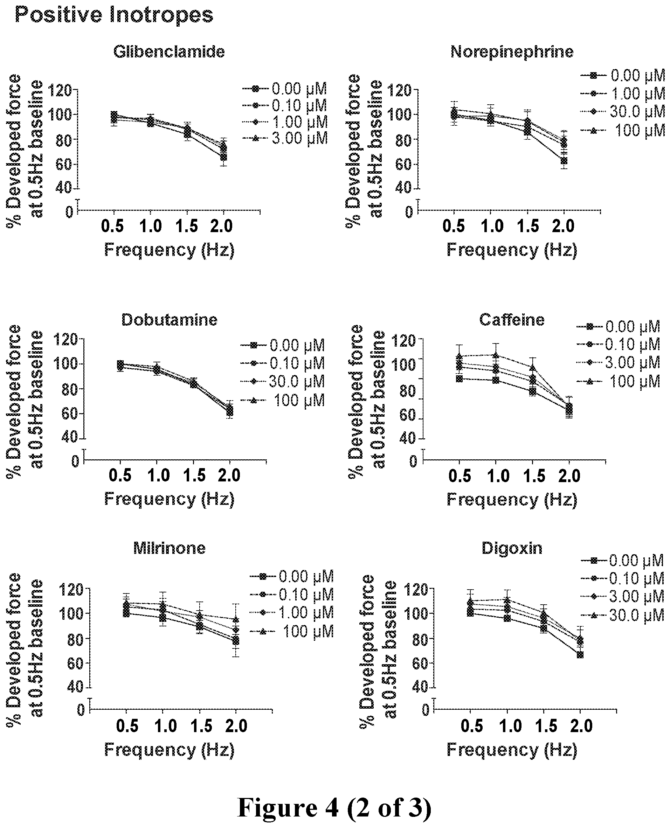

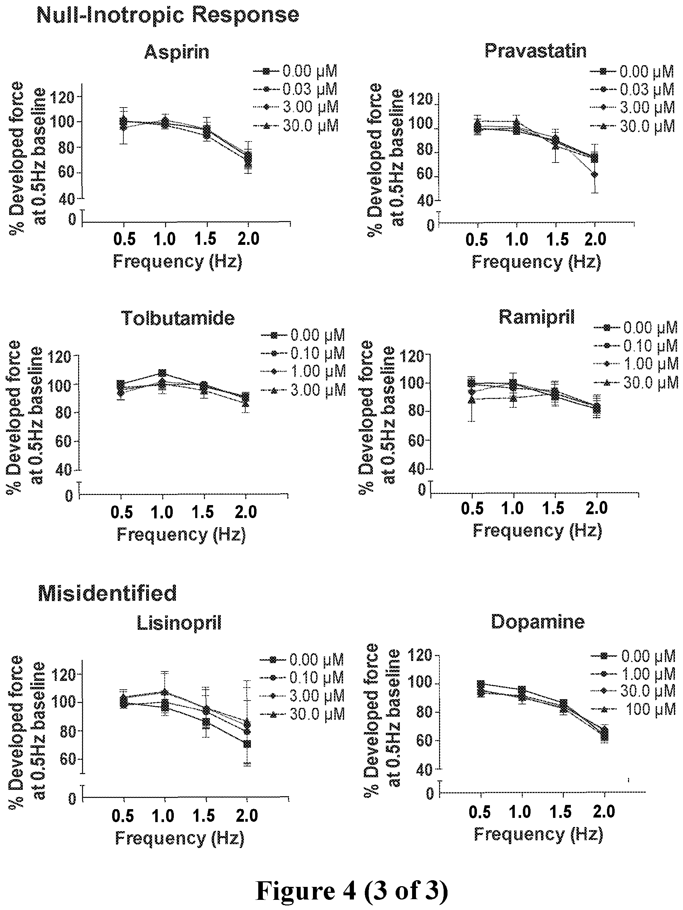

**Figure 4****.** Force-frequency analysis showing contractile force in response to the 17 unknown compounds at increasing dosages within the effective concentration range at all pacing frequencies, normalized to the generated force at 0.5 Hz (30 bpm) in drug-free baseline condition. The unknown compounds were classified into positive inotropes, negative inotropes and compounds with null inotropic response according to the force-frequency analysis. Two (2) out of the 17 compounds were misidentified. Data show mean±SEM of n=3-8.

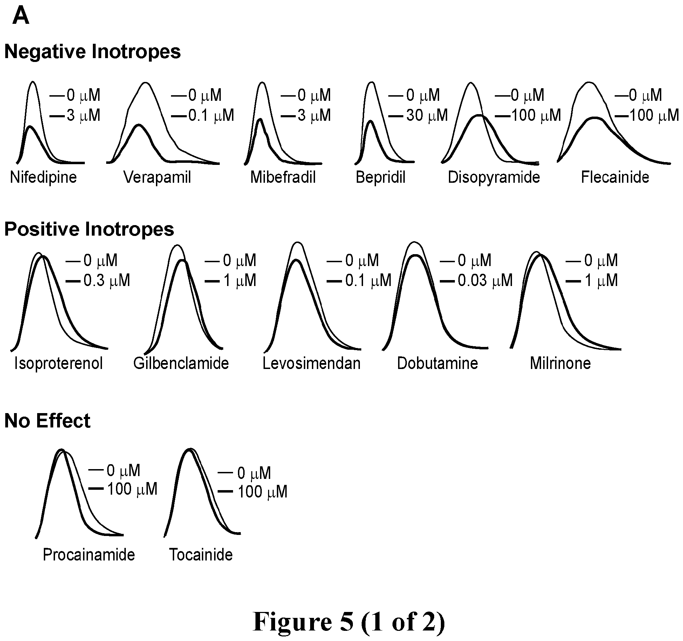

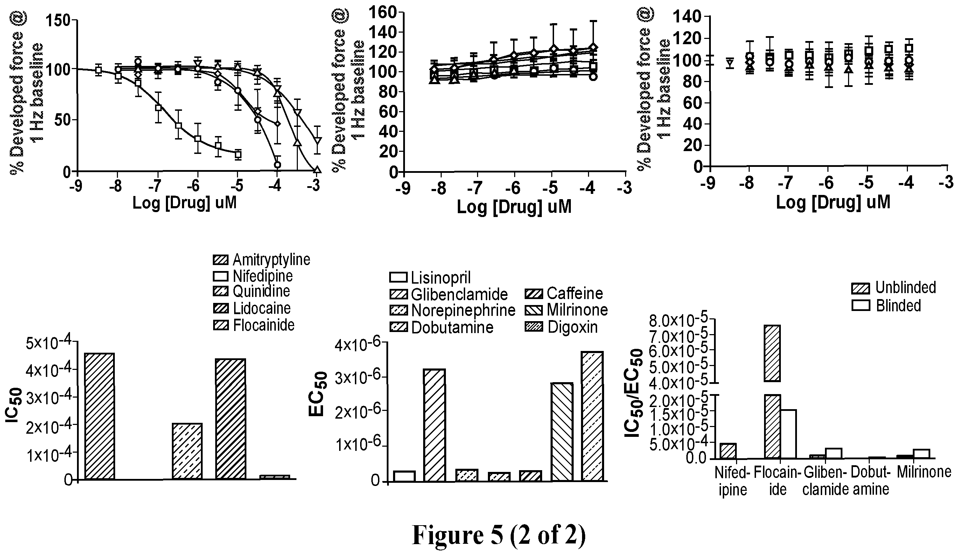

**Figure 5****.** Screening of known inotropic drugs with hvCTS. **A.** Representative tracings showing contraction profiles of known positive inotropes, negative inotropes and drugs with no known inotropic effects. **B.** Dose-response curves fitted at the 1 Hz pacing frequency for drugs showing positive, negative and no inotropic effects. C. IC₅₀ for drugs showing negative inotropic effect and EC₅₀ comparison for drugs showing positive inotropic effects calculated at the 1 Hz pacing frequency. Left panel, left to right: Amitryptyline, Nifedipine, Quinidine, Lidocaine, and Flecainide. Center panel, left to right: Lisinopril, Gilbenclamide, Norepinephrine, Dobutamine, Caffeine, Milrinone, and Digoxin. **D.** Comparison of EC₅₀/IC₅₀ determined from blinded and unblinded screening of inotropes.

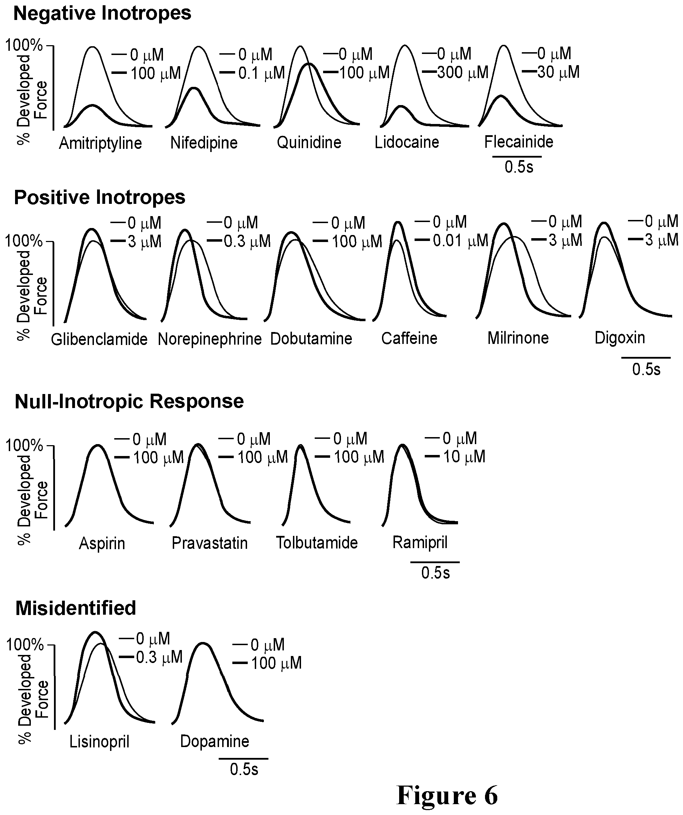

**Figure 6****.** Representative hvCTS twitch force tracings for the 17 compounds screened in the blinded study. The unknown compounds were classified into positive inotropes, negative inotropes and compounds with null inotropic response according to the force-frequency analysis. Two (2) out of the 17 compounds were misidentified.

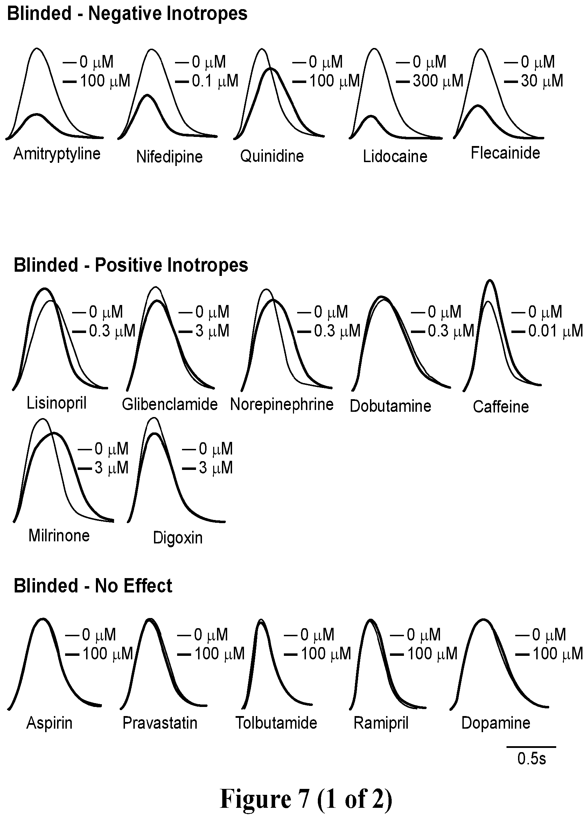

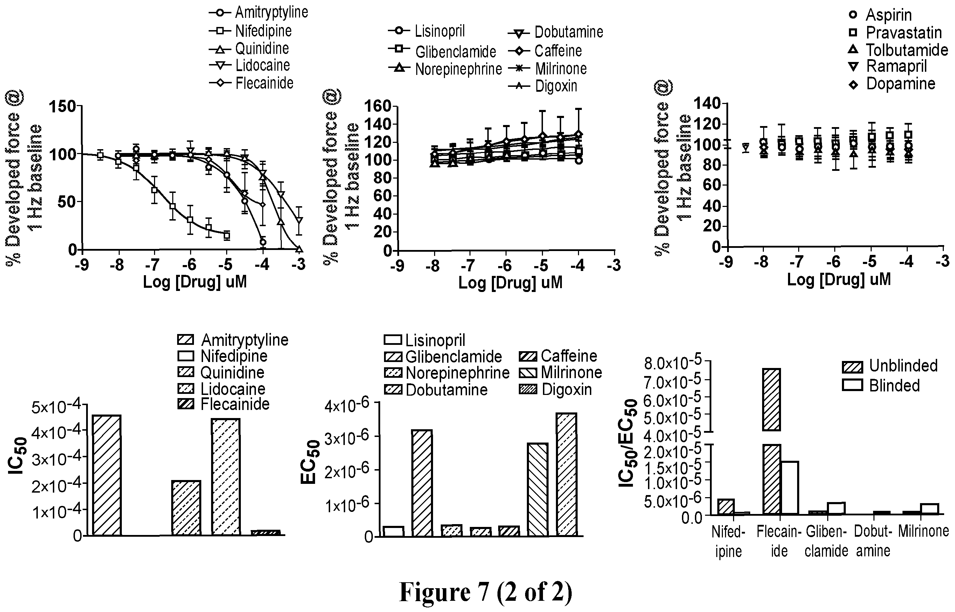

**Figure 7****.** Blinded screening of compounds with hvCTS. **A.** Representative tracings showing contraction profiles of drugs classified as having a negative inotropic, positive inotropic and no effect after data analysis. **B.** Dose-response curves fitted at the 1 Hz pacing frequency for drugs showing positive, negative and no inotropic effects. **C.** IC₅₀ for drugs showing negative inotropic effect and EC₅₀ comparison for drugs showing positive inotropic effects calculated at the 1 Hz pacing frequency. Left panel, left to right: Amitryptyline, Nifedipine, Quinidine, Lidocaine, and Flecainide. Center panel, left to right: Lisinopril, Gilbenclamide, Norepinephrine, Dobutamine, Caffeine, Milrinone, and Digoxin. **D.** Comparison of EC₅₀/IC₅₀ determined from blinded and unblinded screening of inotropes.

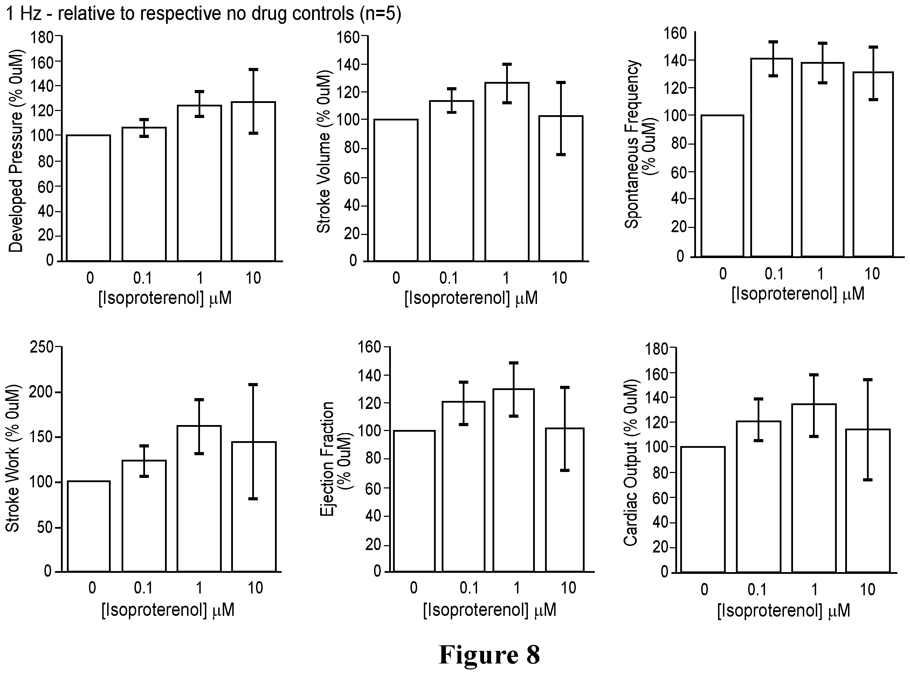

**Figure 8****.** Comparison of drug effects in hvCTS and hvCOC. Effect of isoproterenol on human ventricular cardiac organoid chamber (hvCOC, a 3D cardiac organoid) function shows an increase in stroke work, cardiac output and pressure volume loop. A more robust increase in pressure in hvCOC cardiac organoids was observed relative to the increase in contractile force observed in hvCTS in response to isoproterenol.

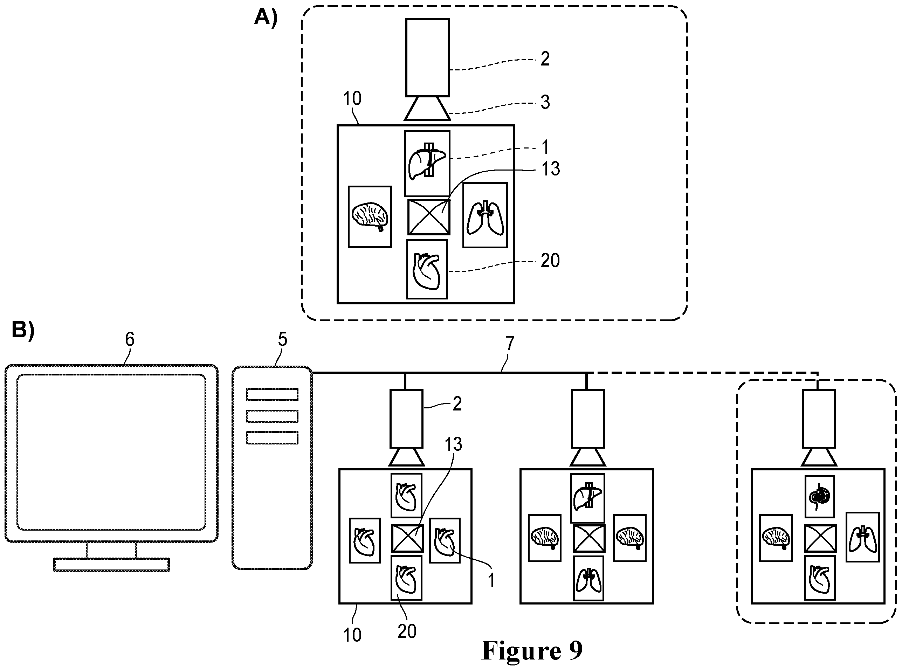

**Figure 9****.** A) A schematic illustration of a bioreactor system comprising an organoid module 10, a computer-controlled detection/recording device 2 (*e.g.,* a camera) for simultaneously imaging up to four organoid cartridges 20 (and optionally saving the images), each containing an organoid 1 (at least one of which is a heart, whereas the others can be any organoid *e.g.*, heart, brain, nerve, liver, kidney, adrenal gland, stomach, pancreas, gall bladder, lung, small intestine, colon, bladder, prostate, uterus, blood, vascular, tumor, eye, or skin), via reflective pyramidal mirror 13. An organoid module 10 may contain a multiple of the same type of organoid 1 or a variety of organoid 1 types. B) A diagram of the imaging bioreactor platform consisting of a computer or data processor 5 controlling an array of organoid modules 10.

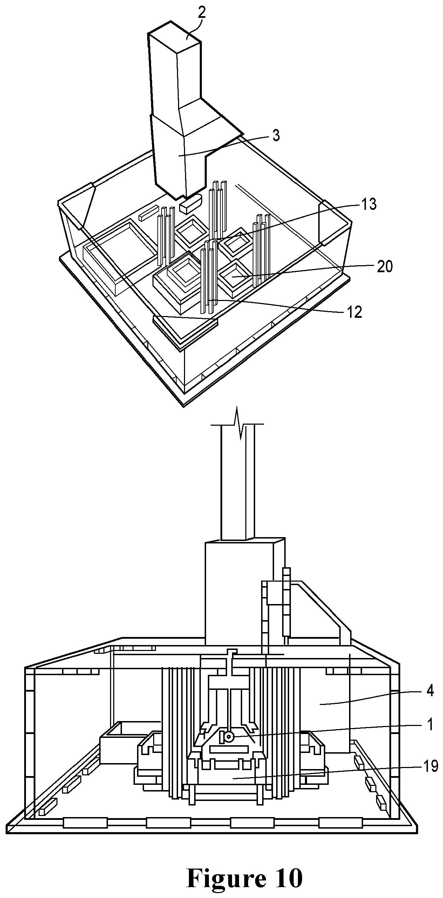

**Figure 10****.** Three-dimensional rendering of an organoid module 10 with four organoid cartridges 20. Isometric and side views are presented. Also shown is an organoid 1, a detection/recording device 2 connected to a lens 3, lights 12 (*e.g.,* LED lights), a pyramidal mirror 13, an organoid cartridge 20, a temperature control element 4 (*e.g.,* a heater), and a mixer 19, such as a stir bar.

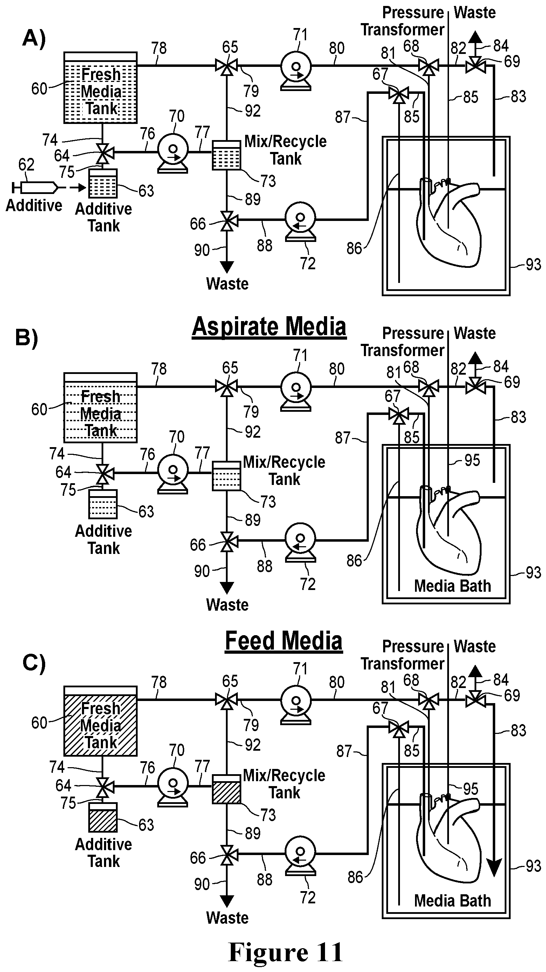

**Figure 11****.** A) Schematic of fluidic exchange system for an organoid cartridge, including fluidic lines, pumps, valves, pressure transducer and fluid tanks. Specific configurations of valves and pumps are used depending on the function, such as B) aspiration or C) fresh media addition to the media bath. A detailed description of the illustrated embodiment of the bioreactor system is presented in Example 6.

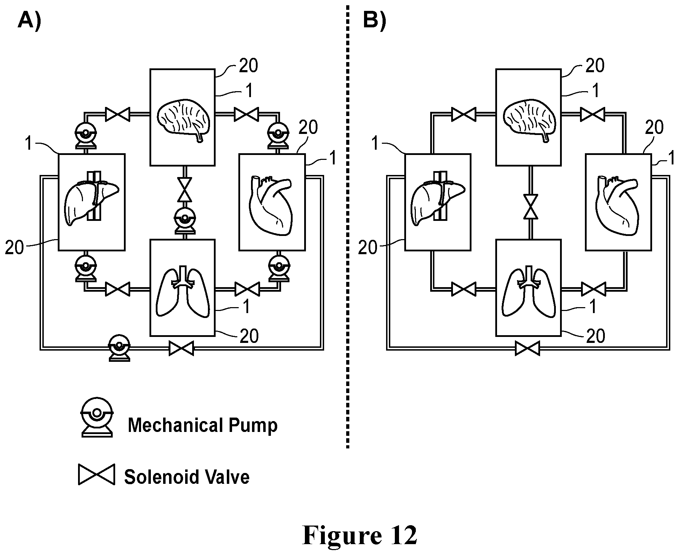

**Figure 12****.** A) Graphical representation of fluidic exchange system consisting of fluidic lines, pumps, and valves that direct media between multiple organoid cartridges within a module. A variety of organoid types can be connected to simulate a "body-in-a-jar". B) A heart organoid with sufficient pumping ability could be utilized as the sole biological pump to form a self-powered "body-in-a-jar". Example 6 provides additional description of these embodiments of the bioreactor system.

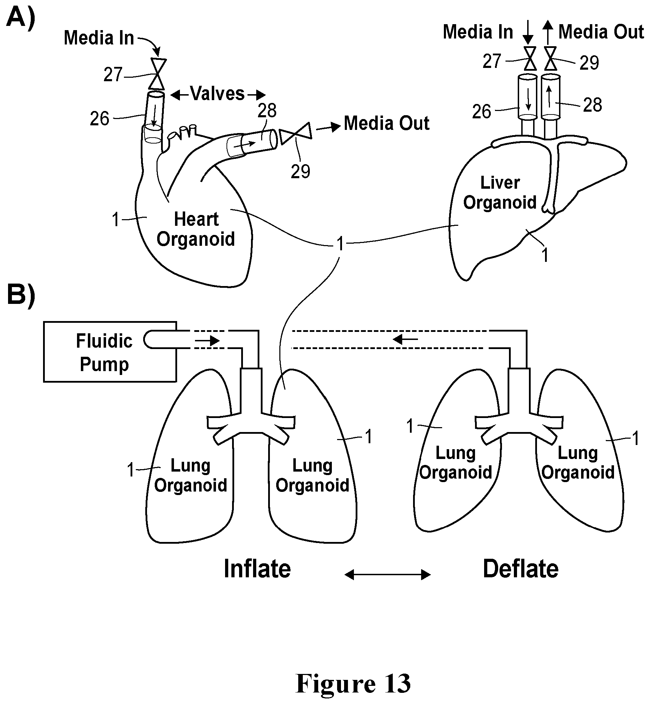

**Figure 13****.** A) An embodiment of the bioreactor system is illustrated that shows inlet and outlet pathways for media exchange through an organoid 1 controlled by valves (left pane: heart organoid; right pane: liver organoid). B) Schematic of mechanical stimulation system where a reversible fluidic pump is connected to an organoid 1 for inflation and deflation. The organoid 1 is subject to stretch based on changes in pressure delivered by the stimulation system and the pliability of the organoid 1.

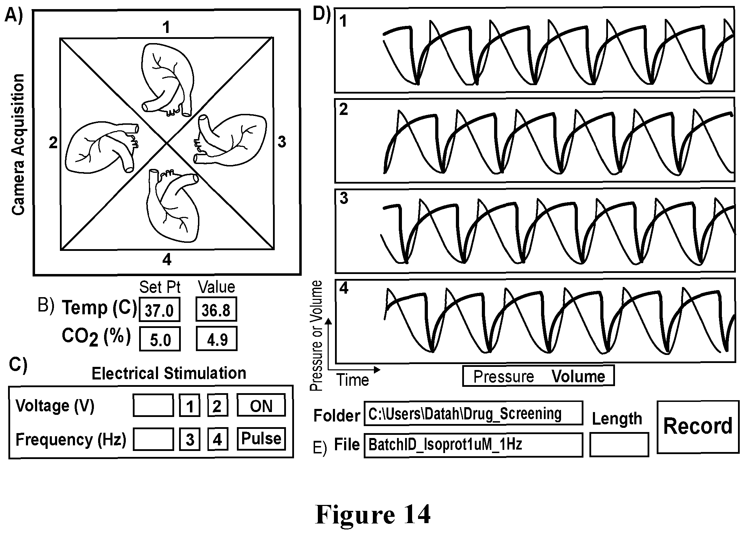

**Figure 14****.** Schematic of LabVIEW front panel for operating the bioreactor platform or system. A) Acquisition preview window of multiple organoids. B) Environmental control panel. C) Electrical stimulation parameters. The user has options to control the voltage power, alter the frequency, select which chambers to stimulate and decide to send continual stimulation or a single pulse. D) Real-time pressure, volume data of four distinct organoids. Pressure is represented as grey lines while organoid volume is represented as black lines. E) Recording parameters.

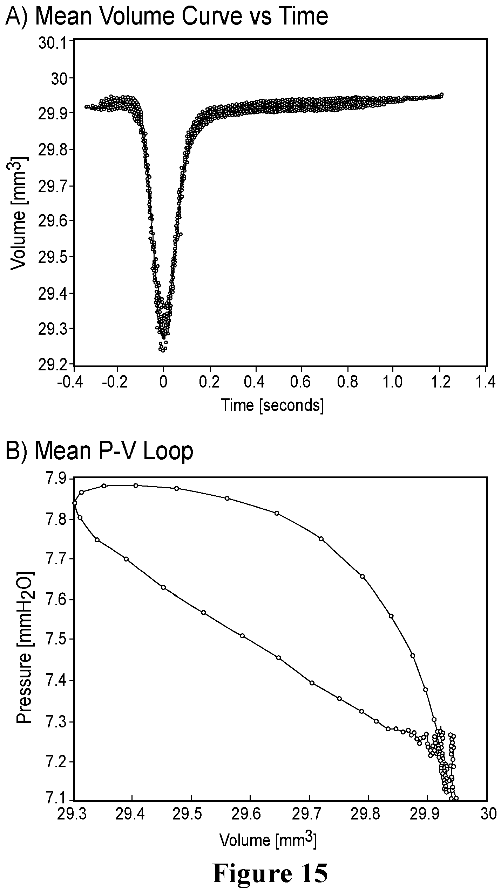

**Figure 15****.** MATLAB analysis to generate average P-V loops from an acquisition. A) Calculation of mean volumetric contraction curve of a tissue. Each volumetric contraction of a beat is plotted as a scatter plot with the time of maximum contraction set to t=0 seconds. Mean curve is denoted as a solid red line. B) Line graph of mean P-V loop summarizing multiple contractions. Red circles denote values at sampled time points.
**Figure 16****.** Flow diagrams of LabVIEW software used to monitor cells, tissues, and organoids in the system and apparatus disclosed herein. Flow diagrams schematically exemplify the software-based control of environmental variables, such as temperature and CO₂ level, and the software-based control of features of the system and apparatus, such as lens control, control of lighting, and control of electrical stimulation of cells, tissues and/or organoids contained in the system or apparatus.

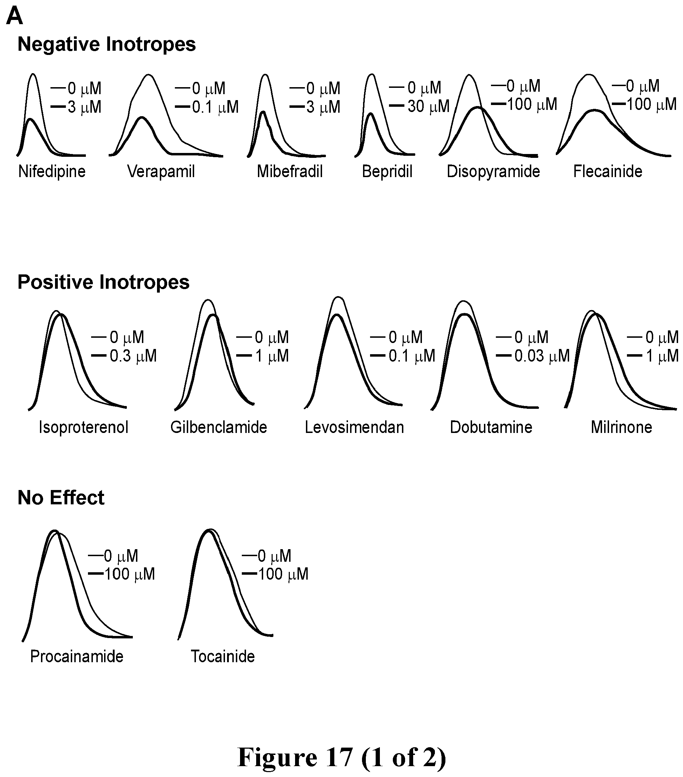

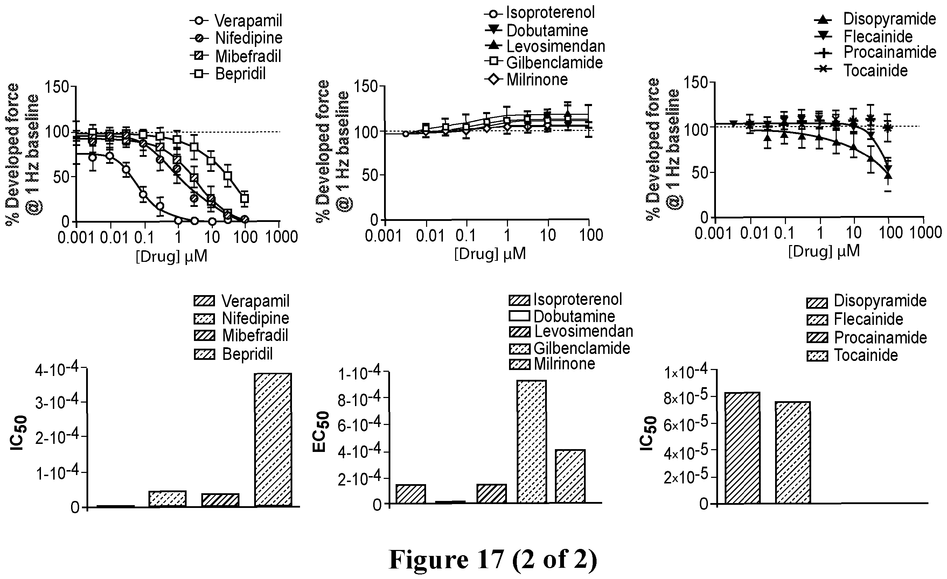

**Figure 17****.** Screening of known inotropic drugs with hvCTS. **A.** Representative tracings showing contraction profiles of known positive inotropes, negative inotropes and drugs with no known inotropic effects. **B.** Dose-response curves fitted at 1 Hz pacing frequencies for positive and negative inotropes. No dose-response relationships were correlated for drugs with no known inotropic effects. **C.** IC₅₀ comparison for negative inotropes and EC₅₀ comparison for positive inotropes calculated at the 1 Hz pacing frequency.

### DETAILED DESCRIPTION

The disclosure provides a multi-tiered system and associated methods for screening compounds for inotropic effects, and can also be used to assess other compound effects on biological cells, tissues and/or organs, such as toxicity. The system and method are effective in identifying and characterizing positive inotropes and/or negative inotropes. A typical configuration involves a two-tiered system and associated method with the first tier designed to provide an accurate yet rapid, versatile, and cost-effective initial screen of compounds for inotropic effects. The first-tiered system comprises a Cardiac Tissue Strip (CTS), such as a human ventricular Cardiac Tissue Strip (hvCTS) supported in a manner that allows for significant flexibility in movement of the gel, with an associated detection (*e.g.,* recording) device to capture gel movement in the presence or absence of a test compound. The CTS is simple to prepare and is used in a straightforward method for screening compounds for the capacity to induce cell-embedded gel movement. The first-tier system and method are amenable to high-throughput formats as well as conventional formats. A second tier screening system and method involves a tissue or organoid developed and maintained in an organoid cartridge typically located in an organoid module, as described herein. The second tier screen involves exposure of a tissue or organoid to a compound, preferably a compound exhibiting inotropic effects, in a cartridge placed in an environment where a detection (*e.g.,* recording) device can monitor organoid behavior. The environment also typically provides for the delivery and removal of fluid such as media and compound-containing fluid under controlled conditions, with various controls needed to maintain an environment compatible with tissue or organoid viability. The two-tiered system is used in a two-tiered method that reveals compounds having inotropic effects at the cellular, tissue and/or organoid or organ level, increasing the accuracy and reliability of results obtained in screens for compounds having inotropic effects. Moreover, the disclosure provides for a three-tier system and method involving the above-described two-tier system and method supplemented by a third-tier system and method involving a multi-organoid (or multi-tissue or mixed tissue and organoid) module system and associated method. In this third tier, multiple tissues and/or organoids, which may be of the same type (*e.g.,* cardiac) or different types (*e.g.,* cardiac and liver), are developed and maintained in distinct organoid cartridges that may conveniently be located in a single organoid module (it is understood that organoid cartridges an organoid modules may contain tissues or organoids). This typical arrangement conveniently allows for a single mirror system such as a pyramidal mirror system, to be used in conjunction with a single detection (*e.g.,* recording) device. In subjecting a compound to the three-tiered system and method, information is obtained about the inotropic effects of the compound on cells as well as the effects of the compound (including inotropic effects) on one or more cognate tissues, organoids or organs, or on a plurality of different tissues, organoids or organs. The three-tiered screening system and method further strengthens the data obtained in terms of accuracy, reliability and reproducibility, with a manageable addition to cost in terms of money and time.

In drug screening methodologies, three-dimensional (3D) engineered tissues have an advantage over two-dimensional (2D) preparations in that 3D shows greater physiological relevance when compared to 2D monolayers, including gene expression and electrophysiology. Consistent with this view, demonstrated herein is the fact that higher order 3D tissues are functionally more mature and more accurately display contractile responses when exposed to known pharmacological agents.

One advantage of the hvCTS in the first tier of the screening system disclosed herein is that contractile properties were measured intact within the PDMS mold, without the need to transfer the hvCTS to another vessel, allowing for a higher degree of standardization and higher throughput while preserving the possibility of long-term experimentation. It is recognized that variations between batches of tissues may exist, and may yield a variance on the order of a factor of 10. Without wishing to be bound by theory, this variation may be due to the variation in geometry and variation in elastic properties of the PDMS posts, which were fabricated manually, leading to variation in the calculation of forces. Inclusion and exclusion criteria have been introduced based on about 200 hvCTS that have been analyzed to objectively and systematically identify outliers, which were less than 2% of the hvCTS fabricated for the study. Specifically, those hvCTS with a developed force of <0.01mN (< 10% percentile) when paced at 1 Hz during baseline, drug-free conditions were excluded from the study.

The hvCTS system comprises a biocompatible gel. The gel may be formed from Matrigel^{®} (*e.g.*, Cultrex BME^{®}) or any biocompatible compound or mixture capable of forming a gel and providing an environment in which cells contained within the gel are able to function in a manner consistent with their native physiological functioning. A biocompatible gel can be formed from agarose, gelatin and other compounds and compound mixtures known in the art, with Matrigel^{®} a preferred gel material because it is known to be conducive to cells expressing *in vivo* physiological functions, including maintenance of a pluripotent developmental state by stem cells. The gelling material, such as Matrigel^{®}, may be present in a range of concentrations, *e.g.*, 2-25 mg/ml, including at least 0.5 mg/ml, with an exemplary concentration being about 1 mg/ml. It is contemplated that the biocompatible gels of the disclosure may comprise compounds in addition to the gelling material, such as Matrigel^{®}. For example, biocompatible gels may be supplemented with collagen, *e.g.,* type I human collagen, and/or other extracellular matrix proteins.

Embodiments of the hvCTS system comprise a biocompatible gel comprising cardiomyocytes, such as human cardiomyocytes (*e.g.,* human ventricular cardiomyocytes). The cardiomyocyte may be derived from cardiac tissue, a heart organ or may be derived from a human pluripotent stem cell. The cardiomyocytes may be present in the biocompatible gel at a range of concentrations, *e.g.*, 10⁴-10⁹ cells/ml, such as a concentration of 10⁶ cells/ml of biocompatible gel. A primary focus of the disclosed system and methods is cardiomyocyte behavior and cardiac physiology, giving rise to "hvCTS" in the name of the disclosed system, the disclosure contemplates biocompatible gels comprising myocytes (*e.g.,* skeletal and smooth muscle myocytes) and other cells amenable to physiological monitoring using the disclosed system. The same biocompatible gel is also suitable for making the cardiac organoid used in the second tier of screening, allowing direct comparison between results attained in the two tiers of screening.

In embodiments of the hvCTS system comprising cardiomyocytes, the biocompatibility of the gel allows the cardiomyocytes to interact in a manner that results in a collective capacity to contract and relax under conditions that lead to that behavior *in vivo.* In particular, the cardiomyocytes contained in the biocompatible gel collectively contract upon electrical stimulation at the pacing frequencies disclosed herein, and the force of contraction can be altered by known inotropes, with known positive inotropes increasing the force of contraction from a given electrical impulse and known negative inotropes decreasing the force of contraction from a given electrical impulse. The electrical impulse may be provided by any suitable power supply capable of delivering an electrical impulse of 10V, duration 5 msec, at a delivery or pacing frequency of 0.1-10 Hz, such as by providing a pacing frequency of 0.5, 1.0, 1.5, and/or 2.0 Hz.

Formation of the biocompatible gel is achieved using a mold of any suitable set of dimensions and made of any material compatible with gelation of the biocompatible gel material, such as Matrigel^{®} comprising human ventricular cardiomyocytes. In some embodiments, the mold is formed of polydimethylsiloxane, which is compatible with Matrigel^{®} gelation and cardiomyocytes. A typical shape of the mold cavity is a rectangular prism (*i.e.,* a three-dimensional rectangle) having a depth (*i.e.,* height or thickness) no less than the expected thickness of the biocompatible gel to be formed in the mold. As disclosed in Example 1, a typical mold cavity can have dimensions of approximately or exactly 26.5 mm x 16 mm x 6 mm, but it is understood that the dimensions of the biocompatible gel, and mold used in the formation thereof, can be scaled up or down, and may vary significantly from the exemplary dimensions disclosed.

The hvCTS system disclosed herein also provides a biocompatible support apparatus for the biocompatible gel. The biocompatible support apparatus allows the biocompatible gel to be disposed in the air, away from any solid-surface support other than the biocompatible support apparatus. The biocompatible support apparatus allows the biocompatible gel to have greater freedom of motion, particularly motion in a vertical plane. A consequence of the greater freedom to move is that any movement of the biocompatible gel arising from contraction can be detected with interference from a support structure reduced or minimized. Accordingly, the biocompatible support structure for the biocompatible gel is at least two vertical support members and, typically, is two vertical support members (*e.g.,* legs or posts). The vertical support members may be composed of any biocompatible material capable of stably supporting the weight of the biocompatible gel. An exemplary material for the support apparatus, *e.g.*, vertical support members, is polydimethysiloxane (PDMS). Any shape compatible with the function of the support apparatus in stably supporting the biocompatible gel is contemplated, with an exemplary shape being at least one pillar having a cross-sectional shape of a circular, an ovoid, a rectangle, a triangle, or at least a five-sided geometric plane form. In embodiments comprising at least two vertical support members as the biocompatible support apparatus, the shape of the vertical support members can be the same, or different. An exemplary biocompatible support apparatus is two approximately circular posts of about 0.5 mm diameter made of PDMS. In general, the height of the vertical support members is similar or the same, resulting in a biocompatible gel that is substantially horizontal, including the appearance of being horizontal to the naked eye. The support apparatus, such as the at least two vertical support members of some embodiments, is affixed to the biocompatible gel by adhesion or mechanical attachment or surface tension, or the support apparatus may be integrated into the biocompatible gel by embedding the support apparatus in the biocompatible gel formulation (*i.e.,* the biocompatible gel material) during gel formation. In combination, the biocompatible gel and the biocompatible support apparatus form a tissue strip, and the tissue strip is a cardiac tissue strip when the cell contained in the biocompatible gel are cardiomyocytes.

The hvCTS system also comprises a detection device, which is any camera, camcorder, film recorder, or digital recording device capable of detecting transient movement of the biocompatible gel. An exemplary recording device is a high-speed camera wherein the camera can record about 100 frames per second, as described in Example 1. In some embodiments, the detection device is in electronic communication with a source of computing power (*e.g.,* a computer) that can record, and optionally analyze, any movement detected by the device. Software capable of conferring the recording and/or analysis functions on, *e.g.,* a computer include motion detection/analysis software sufficiently robust to handle the motion data detected by the detection device. Exemplary software is LabView software.

The disclosure also provides a method of making a cardiac tissue strip by providing a biocompatible mold, such as a PDMS mold, having a cavity of suitable shape and size to form a desired biocompatible gel. The biocompatible gel materials, such as Matrigel^{®} and type I human collagen, are mixed with a plurality of cells such as human ventricular cardiomyocytes and the mixture is added to the mold cavity under conditions where the gel material will undergo gelation. A sufficient quantity of the gel material is added to the mold to cover the base of the cavity and fill the cavity to the height desired for the biocompatible gel. For biocompatible gels comprising Matrigel^{®}, gelation is typically induced by warming the gel materials. The biocompatible support apparatus, such as vertical support members, may be embedded in the gel material prior to gel formation, or the biocompatible support system may be affixed to the form biocompatible gel using adhesion, mechanical attachment, or surface tension.

The hvCTS system is also useful in screens for inotropes, or compounds that alter the force of cardiac contraction. The system is useful in screens for positive inotropes, *i.e.,* compounds that induce an increase in the force of contraction, as well as screens for negative inotropes that decrease the force of contraction. In practice, the hvCTS system with a biocompatible gel comprising cells of interest, *e.g.*, human ventricular cardiomyocytes, is exposed to the leads of a power source supplying 10V, duration 5 msec, at a pacing frequency of 0.1-10 Hz, *e.g.,* 0.5 Hz, 1.0 Hz, 1.5 Hz, or 2.0 Hz. The pulsing is applied to the biocompatible gel with or without contacting the biocompatible gel with a candidate inotrope, and with the detection device operational. In typical embodiments, any movement of the biocompatible gel is detected and analyzed using a source of computing power (*e.g.,* a computer) in electronic communication with the detection device, which is typically a high-speed camera, but may be a camcorder, film recorder or digital detection, and optionally recording, device capable of detecting the movements of the biocompatible gel. If the compound results in altered movement of the biocompatible gel relative to the movement of the gel in the absence of the compound, the compound is identified as an inotrope. If the movement is less in the presence relative to the absence of the compound, the compound is identified as a negative inotrope, and if the biocompatible gel movement is greater in the presence relative to the absence of the compound, the compound is identified as a positive inotrope. The screening methods using the disclosed hvCTS system provide a rapid initial screen for bioactive compounds capable of modulating the force of cardiac contractions. The disclosed methods are suitable for high-throughput screening of compounds and provide a rapid, cost-effective and accurate initial assessment of potential therapeutics. It is contemplated that the methods and system disclosed herein will be used as one stage of a multi-stage development effort to identify cardiac therapeutics, such as by subjecting compounds identified as positive or negative inotropes in the disclosed methods to a second-order screen using, *e.g.,* a cardiac organoid, such as would be found in a cardiac organoid chamber in a system engineered to screen for bioactive compounds.

To confirm the validity and reliability of the hvCTS and hvCOC systems as *in vitro* screening systems for identifying contractile responses to a wide variety of pharmacological compounds, the screening of pharmacological compounds from the major drug classes was conducted in both blinded and non-blinded settings. In the blinded screenings disclosed in the Examples contained herein, all negative inotropes were correctly identified with EC₅₀ estimations that are within the reported range in human ventricular tissue preparations. Moreover, the disclosed hvCTS system was sensitive enough to accurately discern the difference in potency between drugs of the same class, as demonstrated in the case of L-type calcium channel blockers. Responses of the hvCTS system to positive inotropes, including isoproterenol and digoxin, were small, as with other engineered cardiac tissues previously reported [3, 4]. Analyses of drug responses over a range of pacing frequencies at increasing concentrations allowed us to accurately identify those pharmacological compounds that have a reported positive inotropic effect. While the effect of most positive inotropes on the hvCTS is small, where successfully determined, the EC₅₀ determinations were found to agree with known literature values with only 1 out of the 8 tested compounds found to be out of range. Thus, the experimental results reported herein establish that the simple and rapid hvCTS screening system reduces false positives and false negatives. The predictive capacity of the hvCTS screening system was further assessed in a blinded screening, with accuracies for negative, positive and null inotropic effects found to be 100%, 86% and 80%, respectively. Interestingly, the hvCOC screening system, disclosed herein as suitable for the second tier of the two- or three-tiered system, is a screening system with a pro-maturation milieu that yields physiologically complex parameters that displays enhanced positive inotropy. Based on these results, the two-tiered screening system is particularly suited for avoiding false positives and negatives. Using such a screening system will facilitate drug discovery by leading to better overall success, thereby bridging the long-standing gap between inaccurate animal models and human patients. As a consequence, it is expected that the screening systems disclosed herein will lead to better overall screening success and reinvigoration of the drug development pipeline.

Most EC₅₀ values reported for various cardioactive drugs were obtained from isolated strips of cardiac muscles *in vitro,* and the disclosed hvCTS system is comparable to such isolated muscle strips. There are also studies on isolated mouse or rat hearts that measure the cardiac functions of various drugs that have cardiovascular effects. Even with these animal models, however, there are few reported dose ranges with estimations of EC₅₀. The experimental results disclosed herein provide the first EC₅₀ values of a wide range of pharmacological compounds in an assay system based on human cardiac tissue.

The blinded study showed that the first-stage screening has its limits in the detection of positive inotropic effects in compounds, with false positive (lisinopril) and false negative (dopamine) results. With the more mature cardiac organoid, it is expected that positive inotropic effects, dependent on better developed adrenergic signaling and calcium handling, would be more accurately and sensitively detectable. The second-stage screening apparatus provides a platform for simultaneously monitoring one or more human organoids, including at least one cardiac organoid, and providing automated culture, conditioning, signaling between multiple organoids and assessment of a functional human-body surrogate for high-content, species-specific, *in vitro* screening of potential inotropic compounds to enhance preclinical testing of novel therapeutics for efficacy and toxicity, as a preferable alternative to animal testing. Use of a two-stage screening system is expected to yield accurate identification of inotropic compounds in a reliable manner using screening methodology that is rapid and cost-effective. Simultaneous data recording of multiple organoids is expected to greatly improve consistency and throughput, especially with regards to experiments analyzing long-term drug response (*i.e.,* more than 24 hours and up to several weeks). There exists a gap in current *in vitro* therapeutic screening methods that does not account for rare adverse effects. Therapeutic screening is typically done in acute fashion (*i.e.,* within 5-30 minutes post-drug exposure, with brief data acquisition periods) and may inaccurately predict longer-term effects or miss rare events. The two-stage platform also provides a number of features desirable in a screening system, such as rapid, high-throughput initial screening in stage 1, along with potential inotropic compound perfusion, electrical point stimulation, mechanical stimulation, "Plug-and-Play" interchangeable organoid cartridges, and multi-organoid imaging in the second-stage apparatus of the system. Electrical point stimulation of an organoid model allows characterization of conduction properties, unlike typical electrical field stimulation that activates all cells at once. The innovative technology promises to revolutionize the therapeutic discovery process by bridging long-standing obstacles in the therapeutic development pathway.

Human stem cells and their derivatives provide an exciting opportunity for the development of *in vitro* therapeutic screening platforms as they are human-sourced and function similarly to native human cells once differentiated. Standard two-dimensional (2D) cell culture or three-dimensional (3D) embryoid bodies, however, do not replicate essential characteristics of actual human organ tissue. Using tissue engineering, researchers have recapitulated features of human tissue when engineering predictive organoid models. Combining the rapid initial screening of cells contained in a biocompatible gel with the multiple organoid types that can be used in second-stage screening in a single drug screening platform can, and is expected to, further improve the re-creation of human body function and therefore advance our ability to predict therapeutic effects on humans, providing a more effective and ethical alternative to animal testing.

The disclosure provides a modular imaging platform where a data processor (*e.g.,* a computer) simultaneously controls at least one, or a plurality (*i.e.,* multiple), of recording devices (*e.g.,* cameras) wherein each image can record the behavior of a gel comprising cardiomyocytes or can record the behavior of multiple organoids (Figure 9). Figure 16 illustrates software diagrams to achieve such control. An organoid module of the second-stage apparatus is described as an individual bioreactor enclosure for multiple organoid cartridges, with features that aid in therapeutic screening (Figure 10). These features include (a) a mirror arrangement (*e.g.,* a pyramidal mirror) for multi-organoid acquisition using a single recording device, (b) fluidic exchange system for automated media exchange (Figure 11), and (c) regulated exchange of media between organoids (Figure 12). Additional features include (d) a mixer (*e.g.,* a magnetic stirrer) for efficient mixing of media solutions, (e) temperature control element and CO₂ control element to control physiological environmental conditions for long-term culture and acquisition, (f) pressure control for mechanical stimulation during tissue formation and culture (Figure 13B), (g) electrical point stimulation to control beating rate, (h) perfusion for automated delivery of therapeutic agents, and (i) valves to direct fluid through an organoid (Figure 13A).

### Examples

### Example 1

In this Example, the methods used to conduct the studies disclosed in the following Examples are described. Any disclosure relating to human embryonic stem cells that are produced or that have been produced by methods that involve or that have at any point in time involved the destruction of a human embryo is for reference only and is not part of the present invention.

*Derivation of cardiomyocytes from human pluripotent stem cells.* All experiments used, for reference only, cells derived from the human embryonic stem cell line (hESC line) hES2 (ES02, Wicell, Madison, WI, USA) propagated in feeder-free culture in mTeSR1 medium (STEMCELL Technologies) on Matrigel. Directed cardiac differentiation was achieved using a protocol previously developed^{[1]}, with the use of the small molecule Wnt inhibitor IWR-1. The differentiated cardiomyocytes were maintained in differentiation medium (StemPro-34 basal medium (Gibco), StemPro-34 Nutrient Supplement (Gibco), 1X Penicillin-Streptomycin (ThermoFisher), 1X GlutaMAX (ThermoFisher)) without supplements after day 8 until they were used for hvCTS or human ventricular cardiac organoid chamber (hvCOC) creation.

*Generation of hvCTS construct.* On day 14 of differentiation, cell clusters were enzymatically dissociated into single cells with trypsin/ EDTA solution (0.025% trypsin/EDTA; Invitrogen) for 20 minutes and then resuspended in differentiation medium. After 48 hours, the cells were collected by centrifugation (300g, 5 minutes), resuspended in culture medium [DMEM (Gibco) supplemented with 10% newborn calf serum (Gibco), 1X Penicillin-Streptomycin and 25 µg/ml amphotericin B (Sigma-Aldrich)] at a concentration of 1 x 10⁸cells/ml, and combined with a mixture of ice-cold 2mg/ml bovine collagen type I (Sigma-Aldrich) in 0.6X PBS (Sigma), 10µM NaOH (Sigma), 0.8X MEM (Sigma), and 16µM HEPES, and Matrigel (BD Biosciences, San Jose, CA, USA; approximately 10 mg/mL stock concentration), at a cell:collagen:Matrigel ratio of 1:8:1 (v/v/v), and an additional 1 million cells/mL human dermal fibroblasts to aid in tissue compaction. The ratio was optimized for effective generation of contractile force, but concentrations can be adjusted to mimic disease states, for example fibrosis. The cells/collagen/Matrigel mixture was then pipetted into a custom mold made of polydimethylsiloxane (PDMS) (L x W x H = 26.5 mm x 16 mm x 6 mm) elastomer filling the rectangular well with 100 µl (1cm in length) (10⁶ cells/well), and then incubated at 37°C, 5% CO₂ for 2 hours to allow the collagen to polymerize. One of ordinary skill in the art will recognize that the gels can be formed in any of a variety of sizes and shapes, and the molds used to form such gels can also be formed in any of a variety of sizes and shapes, depending on the intended use of the molded gel. The molded mixtures were then bathed with culture medium (DMEM supplemented with 10% newborn calf serum), and maintained in culture with daily half- medium exchanges. Inserts in the casting molds were removed at 48 hours, yielding a self-assembled hvCTS held between 0.5-mm-diameter circular PDMS posts at each end. It is apparent that the posts can be formed from any flexible material amenable to at least one form of sterilization are suitable. As noted above, the whole mold can be scaled up or down depending on the application, and the posts can also be scaled up or down. In general, the height of the posts would be similar to the height of a well (*e.g.,* about 6 mm in an exemplary embodiment). Any of a wide variety of cross-sectional shapes is contemplated as being suitable for use in the system, apparatus and methods according to the disclosure. At 7 days after hvCTS creation, the hvCTS were used for drug screening.

*Drug screening with hvCTS.* Initially, pharmacological compounds were prepared for screening. Compounds for unblinded "open label" screening were purchased from Sigma and stock solutions at the highest possible concentrations were prepared according to the product information. Working concentrations over a 5-logarithmic range were prepared for each drug.

In one blinded screening described herein (*see, e.g.,* Example 4), the powders of 17 compounds were reconstituted in DMSO and prepared according to the information provided by the supplier. Working concentrations over a 5-logarithmic range were prepared for each drug according to the dose-range recommendation provided by the supplier.

*Dose response and force measurement.* New born calf serum containing medium was replaced with DMEM high glucose with HEPES without phenol red for optical measurement of generated force in hvCTS. The optimal volume for these experiments was determined to be 5mL. The integrated flexible PDMS posts were used as force sensors, with the post deflection captured in real-time with a high-speed camera (100 frames/s) and LabView software (National Instruments, Austin, TX, USA), applying a beam-bending equation from elasticity theory to calculate twitch force, as described previously^{[2]}. These measurements were obtained with the hvCTS maintained in the original molds where they were created with the hvCTS bathed in culture medium at 37°C. For each drug, force generated in response to 9 doses were measured with the drugs added to the hvCTS at half log increments in a cumulative manner. For each dose, the force generated at 0 Hz, 0.5 Hz, 1 Hz, 1.5 Hz and 2 Hz electrical pacing with field electrodes were measured.

*Data analysis.* Data were calculated and analyzed by a customized MatLab program. Force frequency curves for each drug were generated by plotting the generated force at 0.5 Hz, 1 Hz, 1.5 Hz and 2 Hz at different concentrations. All forces were normalized to the generated force at 0.5 Hz at the drug-free baseline condition (100%).

Dose-response curves were constructed by plotting the generated force at 1 Hz as a function of the log concentrations of drugs. The force generated under drug-free baseline condition was defined as 100%. The force generated at each drug concentration was then normalized to the generated force under the cognate drug-free baseline condition. The EC₅₀ of each tested drug was estimated by fitting to a four-parameter dose-response equation given as: Y=Bottom + (Top-Bottom)/(1+10 ^{((LogEC}_{50^{-X)*HillSlope)}}), using commercial software (GraphPad Software Inc.). X is the logarithm of drug concentrations; Y is the normalized force generated at 1 Hz pacing frequency; Top and Bottom are the Y values of the top and bottom plateaus of the curve itself; Baseline is the Y value that defines 0% -- maximal inhibition by a standard drug. Hill Slope quantifies the steepness of the dose-response curve (the higher the Hill Slope, the steeper the curve).

*Human ventricular cardiac organoid chamber (hvCOC) generation.* The initial steps of hvCOC fabrication are identical to that described above for hvCTS generation, from the dissociation of cell clusters to the preparation of the cells/collagen/Matrigel mix with 1 million cells/ml of human dermal fibroblasts.

To create the chamber, we followed a procedure similar to a published procedure²⁹. Briefly, the top of a 3 x 3 x 6 cm polystyrene bioreactor was modified to permit 9-gauge hypodermic tubing (Small Parts) to pass through the center and 4 mm diameter graphite electrodes (GraphiteStore) to flank the chamber on the edges of the bioreactor. A 6-Fr silicone balloon Foley catheter (Cook Medical), with the tip cut off and sealed with silicone caulking, was threaded through the 9-Gauge tubing into the center of the bioreactor. A small ring cut from hydrophilic porous polyethylene (Fisher Scientific) was placed just above the balloon on the hypodermic tubing.

The balloon was filled with distilled water to the desired chamber size and positioned concentrically within a 2% agarose mold created in phosphate-buffered saline (PBS) and made by inserting a 13 mm diameter test tube (VWR) into the center of the agarose. The agarose mold forms the outer mold boundary while the catheter balloon forms the inner boundary. The catheter was gently pulled against the hypodermic tubing and clamped above the tubing with a nylon screw compressor clamp (Fisher Scientific) to ensure that the balloon did not move within the mold. One mL of the ice-cold sterile tissue mix was transferred to the mold, ensuring complete coverage of the porous polyethylene ring. The entire device was incubated for 2 hours at 37°C and 5% CO₂ to initiate gel polymerization, then immersed in NCS culture media. After 24 hours, the top of the bioreactor, with the tubing, catheter and tissue, was removed from the agarose and transferred to a second polystyrene bioreactor containing 20 mL of NCS media. The tissue was maintained in this environment for 10 days at 37°C and 5% CO₂, with daily half-media changes, during which time the tissue compacted around the balloon core to form a human engineered cardiac organoid.

*Characterization of hvCOC contractile function.* After 10-12 days in culture, the silicone balloon was deflated and carefully removed from the inside of the organoid. A high-sensitivity differential pressure transducer (Millar) was inserted into the lumen of the chamber through a closed loop fluid system connected to a large reservoir of media. A digital camera (PixelLINK) mounted outside of the bioreactor permitted direct tissue monitoring with images acquired at 43 frames/second. Chamber pressure and digital video were acquired simultaneously using a custom acquisition program built in LABVIEW (National Instruments) and chamber cross-sectional area was extracted from the video using a custom script in MATLAB (MathWorks). Chamber volume was estimated by assuming an equivalent sphere with the same cross-sectional area. Functional pump analysis was performed under field stimulation at 63 mV/mm with a GRASS S88x stimulator (Astro-Med) at specified frequencies. Stimulation pulse length was 50 ms. Both pressure and video signals were passed through a digital low pass filter with a 13 Hz cut-off frequency in MATLAB prior to data analysis.

### Example 2

*Development of hvCTS-based drug screening protocol.* To validate hvCTS as a model for drug screening, hvCTS were subjected to 0 Hz, 0.5 Hz, 1 Hz, 1.5 Hz and 2 Hz electrical pacing. Results showed a flat-to-positive force-frequency relationship from 0.5 Hz to 1 Hz and a negative force-frequency relationship from 1 Hz to 2 Hz, with a decrease in developed contractile force at increasing pacing frequencies (Figures 2 and 3). When the negative inotrope nifedipine was administered to the hvCTS, a dose-dependent decrease in developed contractile force was observed at all pacing frequencies tested (Figures 2C, 2D, and 3). The IC₅₀ determined at 0.5 Hz, 1 Hz, 1.5 Hz and 2 Hz were 2.3 µM, 0.62 µM, 0.61 µM and 0.54 µM, respectively. When the positive inotrope isoproterenol was administered to the hvCTS, a dose-dependent increase in developed contractile force was observed at all pacing frequencies tested (Figures 2C and 2D). The EC₅₀ determined at 0.5 Hz, 1 Hz, 1.5 Hz and 2 Hz were 0.039 µM, 0.059 µM, 0.054 µM and 0.031 µM, respectively. No consistent change in developed contractile force was observed at the different frequencies tested for tocainide. As there was no significant difference in the EC/IC₅₀ determined at different pacing frequencies and a positive force-frequency relationship was only observed from 0.5 Hz to 1 Hz, the EC/IC₅₀ was determined at the 1 Hz pacing frequency for subsequent screenings after the force-frequency relationships for all concentrations tested for the drug were analyzed to determine the dose-dependent effect on developed contractile force in the tested hvCTS.

### Example 3

*Screening of known pharmacological agents affecting cardiac contractility.* As the next validation step, drugs with known effects on cardiac contractility were tested. A total of 13 drugs with known positive, negative or no effect on cardiac contractility were tested. The hPSC-CMs used for fabrication of hvCTS used in the study had an average purity of 80±2.2% cTnT+ cells. At baseline, 65.7% (n=70) of the hvCTS developed spontaneous contraction. All hvCTS developed contractile force upon electrical stimulation. The average developed contractile force when electrically paced at 1 Hz was 0.076±0.025 mN (n=70). Those hvCTS with a developed force of less than 0.01mN (less than 10% percentile) when paced at 1 Hz during baseline drug-free conditions (see Table 1) were excluded from the study.

Calcium-channel blockers, including verapamil, nifedipine, bepridil and mibefradil, all showed a dose-dependent decrease in developed contractile force in hvCTS tested with IC₅₀ calculated at the 1 Hz pacing frequency with exposure to the calcium-channel blocker at 0.078 µM, 4.2 µM, 3.3 µM, and 38 µM, respectively (Figures 3, 17B, and 17C). Two Class I antiarrhythmics, *i.e.,* disopyramide and flecainide, also showed a dose-dependent decrease in developed contractile force with a considerably higher EC₅₀ of 80 µM and 75 µM, respectively (Figures 3, 17B, and 17C), while two other Class I antiarrhythmics, *i.e.,* procainamide and tocainide, did not show any effect on developed contractile force at all concentrations and pacing frequencies tested (Figure 17B). Beta agonists, including isoproterenol and dobutamine, which are known positive inotropes, showed a small but consistent dose-dependent increase in developed contractile force at all tested pacing frequencies with an EC₅₀ of 0.15 µM and 0.015 µM, respectively, with contractile forces measured using a 1 Hz pacing frequency (Figures 17B and 17C). Other known positive inotropes, including the calcium sensitizer levosimendan, the K_{ATP} channel inhibitor glibenclamide, and the phosphodiesterase inhibitor milrinone, also showed a dose-dependent increase in developed contractile force at all pacing frequencies with EC₅₀ in the sub-micromolar range (levosimendan = 0.15 µM; glibenclamide = 0.93 µM; milrinone = 0.41 µM) (Figures 17B and 17C).

**Table 1: Comparison of EC₅₀/IC₅₀ values for tested compounds measured with hvCTS assay and as reported in the literature.**

| **Drug** | **-log EC₅₀ in hvCTS (EC₅₀ in brackets)** | **Relevant concentration in human/ experimental setting** | **References** |
|---|---|---|---|
| Amitriptyline | Blinded: 3.35±3.13 (450 µM) | 98-208 ng/mL (0.35-0.74 µM) [5], plasma concentration in psychiatric patients | [5], [6] |
| | | 40-400 µM [6], 30-60% effective concentration in isolated human atrial tissue from cardiac bypass | |
| Aspirin | No effect | | [7] |
| Bepridil | Unblinded: 4.42±1.46 (37.9 µM) | 100 µM [8], human atrial and ventricular cardiomyocytes | [8] |
| Caffeine | Blinded: 6.54±0.59 (0.287 µM) | 20 mM [9], in chick embryonic ventricular cells | [9] |
| Digoxin | Blinded: 5.44±0.34 (3.65 µM) | 1 ng/ml (1 nM) ( [10], increased pre-ejection period index | [10] |
| Disopyramide | Unblinded: 4.09± 9.6 (82.2 µM) | 824 µg [11], EC₅₀ of developed tension in canine papillary muscle | [11] |
| Dobutamine | Blinded: 6.61± 1.51 (0.25 µM) | 147-152 ng/ml (0.487-0.5 µM) [12], peak plasma concentration with increased cardiac output 0.217 µM [13], human atrial trabeculae | [12], [13] |
| | Unblinded: 7.82±1.66 (0.015 µM) | | |
| Dopamine | Undetermined | 20 µM [14], in rat atrial trabeculae | [14] |
| Flecainide | Blinded: 4.82± 0.38 (151 µM) | 296 ng/ml (0.071 µM) [15], peak plasma concentration with decreased LVEF in normal subject | [15, 16] |
| | Unblinded: | 500 µg/L (1.2 µM)[16], single 300 mg oral dose | |
| | 4.12±1.66 (75 µM) | | |
| Glibenclamide | Blinded: 5.50± 1.03 (3.17 µM) | 479 ng/ml (0.096 µM)[17], peak plasma concentration after 5 mg oral administration for improved ejection fraction and work product | [17] |
| | Unblinded: 6.03±1.20 (0.93 µM) | | |
| Isoproterenol | Unblinded: 6.83±0.46 (0.15 µM) | 0.8 µM [18], non-failing human myocardium 5.4 nM [3], drug screening with EHT from hiPSC-CMs | [18], [3] |
| Levosimendan | Unblinded: 6.84± 1.03 (0.15 µM) | 0.19 µM [13], human atrial trabeculae | [13] |
| Lidocaine | Blinded: 3.36±0.80 (439 µM) | 20 µM, [19] negative inotropic effect in sheep cardiac Purkinje fibers | [19] |
| Lisinopril | No effect | | [20] |
| Mibefradil | Unblinded: 5.5± 0.28 (3.39 µM) | 24.5 µM [21], IC₅₀ in human right atrial trabeculae | [21] |
| Milrinone | Blinded: 5.56±1.05 (2.77 µM) | 352.3 ng/ml (0.166 µM) [22], bolus 50 µg/kg intravenous infusion for improved cardiac index 228 ng/ml, [22] continuous intravenous infusion at 0.5 µg/kg/minute for improved cardiac index | [22] |
| | Unblinded: 6.39±0.78 (0.41 µM) | | |
| Nifedipine | Blinded: 6.81±0.25 (0.15 µM) | 29-73 ng/ml (0.083-0.21 µM) [23], plasma concentration in patients with ischemic heart disease | [23] |
| | Unblinded: | 0.112 µM [21], IC₅₀ in human right atrial trabeculae | |
| | 5.37±2.2 (4.3 µM) | | |
| Norepinephrine | Blinded: 6.46±0.81 (0.35 µM) | 267 pg/ml (0.15 nM) [24], plasma level after epinephrine infusion | [24] |
| Pravastatin | No effect | | [25] |
| Procainamide | Undetermined | 7952 µg [11], EC₅₀ of developed tension in canine papillary muscle | [11] |
| Quinidine | Blinded: 3.69±0.14 (0.20 mM | 2.07 µg/ml (6.3 µM) [26], peak plasma concentration at 495 mg | [26] |
| Ramipril | No effect | | [27] |
| Tocainide | Undetermined | 1212 µg [11], EC₅₀ of developed tension in canine papillary muscle | [11] |
| Tolbutamide | Undetermined | 63.5 µg/ml (230 µM), [17]peak plasma concentration after 1250 mg oral administration for improved ejection fraction and work product | [17] |
| Verapamil | Unblinded: 7.12±0.14 (0.077 µM) | 97-575 ng/ml (0.21-1.2 µM) [23], plasma concentration in patients with ischemic heart disease | [23], [15], [21], [28] |
| | | 0.61 µM [28], hECT in muscle bath | |
| | | 0.9 ng/ml (0.2 nM)[15], peak plasma concentration with increased systolic time interval | |
| | | 0.123 µM [21], IC₅₀ in human right atrial trabeculae | |

Analysis of the baseline data obtained from open-label, unblinded drug screening assays is presented in summary form in Table 2.

**Table 2: Baseline data of developed force, spontaneous frequency and purity of hPSC-CMs used in hvCTS used in open label unblinded drug screening assay**

| | **DEVELOPED FORCE @ 1HZ** | **SPONTANEOU S FREQUENCY** | **% CTNT+ (BY BATCH)** |
|---|---|---|---|
| NUMBER OF VALUES | 70 | 70 | 22 |
| | | | |
| MINIMUM | 0.0020 | 0.0 | 63.70 |
| 25% PERCENTILE | 0.0130 | 0.0 | 70.60 |
| MEDIAN | 0.0245 | 0.3154 | 83.05 |
| 75% PERCENTILE | 0.0505 | 0.5019 | 89.30 |
| MAXIMUM | 1.269 | 1.356 | 95.20 |
| | | | |
| 10% PERCENTILE | 0.0090 | 0.0 | 64.71 |
| 90% PERCENTILE | 0.1213 | 0.7667 | 93.35 |
| | | | |
| MEAN | 0.07592 | 0.3383 | 80.62 |
| STD. DEVIATION | 0.2110 | 0.3391 | 10.46 |
| STD. ERROR OF MEAN | 0.02522 | 0.04053 | 2.229 |

### Example 4

*Blinded screening of pharmacological compounds affecting cardiac contractility.* A blinded screening of drugs was performed to investigate the effects of the drugs on cardiac contractility. A total of 17 drugs with known positive, negative or no effect on cardiac contractility, but whose identities were not known at the time of testing, were tested using the hvCTS screening protocol as described above. The average developed contractile force when electrically paced at 1 Hz was 0.060±0.005 mN (n=128). Upon unblinding of the drug identities, it was found that of the 17 drugs screened, amitriptyline, nifedipine, quinidine, lidocaine and flecainide showed a dose-dependent decrease in developed contractile force at all tested pacing frequencies and were hence correctly identified as having a negative inotropic effect (Figure 7B; see also, Figures 4, 5, and 6). The IC₅₀ values determined at 1 Hz were 0.45 mM, 0.15 µM, 0.20 mM, 0.44 µM and 15 µM, respectively (Figures 5C and 7C). Based on force-frequency analyses at all concentrations, glibenclamide, norepinephrine, dobutamine, caffeine, milrinone and digoxin were correctly identified as having a positive inotropic effect on cardiac contractility (Figures 4, 5B, 6, and 7B). The EC₅₀ values determined at the 1 Hz pacing frequency were 3.2 µM, 0.35 µM, 0.25 µM, 0.29 µM, 2.8 µM and 3.7 µM, respectively (Figures 5C and 7C). Dopamine, a catecholamine that is also a β-agonist and hence possesses positive inotropic effect, did not significantly affect the developed contractile force in our hvCTS system. Four other drugs, including aspirin, pravastatin, tolbutamide and ramipril, which possess no known inotropic effect, did not show any dose-dependent effect on contractility in the hvCTS tested (Figures 4, 6 and 7B). Interestingly, lisinopril, which has no known effect on contractility, showed a small increase in developed contractile force with increasing concentrations, and was therefore classified as having a positive inotropic effect. Overall, the blinded screening with the hvCTS screening protocol and system disclosed herein yielded an overall predictive capacity of 0.76, with a sensitivity of 0.78 and specificity of 0.76.

Analysis of the baseline data obtained from blinded drug screening assays is presented in summary form in Table 3.

**Table 3: Baseline data of developed force and purity of hPSC-CMs used in hvCTS used in blinded drug screening assay**

| | **DEVELOPED FORCE @ 1HZ** | **% CTNT+ (BY BATCH)** |
|---|---|---|
| NUMBER OF VALUES | 128 | 30 |
| MINIMUM | 0.0020 | 38.60 |
| 25% PERCENTILE | 0.01869 | 61.30 |
| MEDIAN | 0.0445 | 73.45 |
| 75% PERCENTILE | 0.0875 | 85.83 |
| MAXIMUM | 0.2700 | 92.20 |
| 10% PERCENTILE | 0.007918 | 42.19 |
| 90% PERCENTILE | 0.1338 | 87.88 |
| MEAN | 0.05951 | 71.46 |
| STD. DEVIATION | 0.05326 | 16.68 |
| STD. ERROR OF MEAN | 0.004707 | 3.045 |

### Example 5

*Second tier drug screening protocol with hvCOC.* Since the increase in developed contractile force of hvCTS in response to the screened compounds with known positive inotropic effect is small, the effect of these positive inotropes was investigated in higher order three-dimensional (3D) constructs, *i.e.,* hvCOC, which are significantly larger in size than the hvCTS disclosed herein (*e.g.,* 10 million versus 1 million cardiomyocytes) and is of a pump-like conformation as opposed to the trabecular muscle-like conformation of the hvCTS. The hvCOC constructs subjected to isoproterenol treatment elicit a concentration-dependent increase in stroke volume, cardiac output, and developed pressure (Figure 8). When comparing the maximal effect obtained at 10 µM isoproterenol, hvCOC elicited 132% of baseline pressure versus 113% of baseline pressure for hvCTS (Figure 8).

### Example 6

*Bioreactor.* The disclosure provides a custom bioreactor used to culture at least one, and in some cases a variety of, tissue-engineered human organoids. In some embodiments, the bioreactor is used as a second-tier screening apparatus, as disclosed herein. The device was designed to allow interconnection and simultaneous measurement of multiple organoids, with features that enhance reproducibility and efficiency in organoid function testing by enabling subsequent characterizations to be performed within the same bioreactor with minimal manipulation or intervention by the operator.

Figure 9 provides a high-level schematic view illustrating the versatility of the disclosed bioreactor system. Figure 9A shows an organoid module 10 which contains at least one organoid cartridge 20. An organoid cartridge contains a single organoid 1 of any type (*e.g.,* heart, brain, nerve, liver, kidney, adrenal gland, stomach, pancreas, gall bladder, lung, small intestine, colon, bladder, prostate, uterus, blood, vascular, tumor, eye, or skin, and the like). An organoid module 10 may contain multiple organoid cartridges 20, and thus may contain multiples of a single type of organoid 1 or a variety of organoids 1. The organoid module 10 is oriented such that a detection/recording device 2, *e.g.,* a camera, can detect and record the contents of the organoid module 10, such as by having a face of the organoid module 10 closest to detection/recording device 2, and preferably perpendicular to the device, be substantially or completely transparent to at least one wavelength of the electromagnetic spectrum detected by detection/recording device 2. Figure 9B presents a data processor 5, *e.g.,* a computer, in connection with at least one organoid module 10. The organoid modules 10 are typically in 1:1 correspondence with the detection/recording devices 2, and the detection/recording devices 2 are in electronic communication with data processor 5 via communication path 7, *e.g.,* either conventional electrical wiring or by wireless communication. Video monitor 6 may also be connected to data processor 5 via communication path 7.

Figure 10 presents a perspective view of an organoid module 10. Within organoid module 10 is located at least one organoid cartridge 20. Disposed within, or without (not shown), organoid module 10, and disposed within (not shown), or without, organoid cartridge 20 is mixer 19, such as a movable platform (*e.g.,* shaker or rotating platform) on which organoid module 10 is placed or a magnetic stirring device (*e.g.,* stir bar) located inside or outside organoid cartridge 20. In some embodiments, located within organoid module 10 is at least one light source 12 for illuminating organoid 1. Also located within organoid module 10 is at least one mirror 13 for directing electromagnetic radiation in the form of direct and/or reflected light images from organoid 1 to detection/recording device 2. In some embodiments, mirror 13 is a pyramidal mirror 13 to direct images from multiple organoid cartridges 20 to a single detection/recording device 2. A pyramidal mirror 13 can join the images of multiple organoid cartridges 20 into a single condensed viewpoint to maximize image resolution, while permitting the individual organoid cartridges 20 to be spaced physically apart from each other.

Figure 11 illustrates elements of an embodiment of the bioreactor system that are involved in fluid movements, *e.g.*, media flow, particularly the fluid movements involved in adding, or feeding, fresh media and removing, or aspirating, spent, or waste, media. Figure 11A illustrates the entirety of the fluidic exchange system for a single organoid cartridge 20 in organoid module 10, with Figure 11B providing the combination of activated valves and pumps for aspiration and Figure 11C providing the combination for feeding fresh media. The components of the system involved in fluid, *e.g.,* media, movements can be located within, or without, organoid module 10. In describing an embodiment of the bioreactor system providing fluid movements as illustrated in Figure 11, attention will be focused on Figure 11B for media aspiration and on Figure 11C for feeding of media to cells, tissues and organoids of the disclosure. It is understood that the combined descriptions of aspiration and feeding will provide a description of the complete fluid communications within an embodiment of the bioreactor system, as illustrated in Figure 11A. In the remaining description of Figure 11, attachments are to be understood as providing fluid communication between the attached components.

Turning now to the features of Figure 11B involved in one embodiment of the bioreactor system for aspirating media, media 93 is in contact with cartridge media-junction D tubing 86, which is attached to junction D valve 67. Also attached to junction D valve 67 is organoid-junction D tubing 85. In addition, junction D valve 67 is attached to junction D-pump C tubing 87, which is attached to pump C 72. Pump C 72 is attached to pump C-junction C tubing 88, which is attached to junction C valve 66. Junction C valve 66 is attached to junction C-mix/recycle tank tubing 89, which in turn is attached to mix/recycle tank 73. In some embodiments, media 93 is recycled and routed towards mix/recycle tank 73. Junction C valve 66 is also attached to junction C-waste tubing 90 leading from junction C valve 66 to waste.

In operation, the feeding of cells of the organoid, involves components highlighted in Figure 11C, including fresh media tank 60, which is attached to fresh media-junction B tubing 78, which in turn is attached to junction B valve 65. Junction B valve 65 is attached to junction B-pump B tubing 79, which is attached to pump B 71. Pump B 71 is in turn attached to pump B-junction E tubing 80, which is attached to junction E valve 68. Junction E valve 68 is also attached to junction E-junction F tubing 82, which is attached to junction F valve 69. Also attached to junction F valve 69 is cartridge media-junction F tubing 83, which also contacts cartridge media 93.

Additional attached components are described that provide fluid communication within the system and permit additional functions, including but not limited to, therapeutic additive dilution, perfusion of therapeutic(s), therapeutic washout of organoid, rinsing of fluidic lines, and the like. Fresh media tank 60 is attached to, and in fluid communication with, fresh media-junction A tubing 74, which in turn is attached to, and in fluid communication with, junction A valve 64, *e.g.,* a three-way fluid controller or valve. Additive container 62, *e.g.,* a therapeutic container, is used to deliver at least one therapeutic to additive tank 63, which is attached to additive tank-junction A tubing 75, which in turn is attached to junction A valve 64. Junction A valve 64 is also attached to junction A-pump A tubing 76, which is attached to pump A 70. Pump A 70 is attached to pump A-mix/recycle tank tubing 77, which is in turn attached to mix/recycle tank 73. In some embodiments, media from fresh media tank 60 is used to dilute therapeutic(s) from additive tank 63 within mix/recycle tank 73. Mix/recycle tank 73 is also attached to mix/recycle tank-junction B tubing 92, which in turn is attached to junction B valve 65. Junction E valve 68 is attached to junction E-organoid cartridge tubing 81. In some embodiments, media can be delivered through junction E-organoid cartridge tubing 81 to increase pressure within organoid 1. A pressure probe, *i.e.,* pressure transducer 95, detects pressure, and changes in pressure, within an organoid 1 and converts the pressure to an analog electrical signal that is typically transmitted to the data processor, thereby allowing pressure to be monitored and adjusted by the system. In addition, the device provides for the washout or rinsing of fluidic lines. In particular, junction F valve 69 is attached to junction F-waste tubing 84, which in turn leads from junction F valve 69 to waste. In some embodiments, fluid can be removed from the fluid exchange system without coming in contact with organoid cartridge 20 by exiting to waste through junction F-waste tubing 84.

Figure 12 presents a higher-level schematic of fluidic exchange within organoid module 10 to illustrate the creation of a "body-in-a-jar". Figure 12A presents a fluidic exchange system that transfers media between at least two organoid cartridges 20 within organoid module 10. Fluid is directed through the system by a series of valves and pumps. Figure 12B illustrates a fluidic exchange system where fluid is directed by valves and is pumped solely by a biological pump (*e.g.,* a heart organoid 1), thereby providing a self-powered "body-in-a-jar".

Figure 13 presents methods of flowing fluid into and out of organoids 1. Figure 13A illustrates an organoid 1 (left panel: heart organoid; right panel: liver organoid) connected to media inlet tube 26 and media outlet tube 28, which permits fluid to be directed into the void of the organoid 1 and out through media outlet tube 28 to a waste path. The direction of fluid flowing through the organoid 1 is controlled by inlet valve 27 and outlet valve 29. Figure 13B represents a method of applying mechanical pressure to an organoid 1, such as a lung organoid 1. A fluidic pump controls fluid flow (*e.g.,* gas or liquid) to organoid 1 and modulates the pressure of the organoid cavity to control the size of organoid 1. Absolute pressure values depend on the material properties of the organoid 1 and the desired size of the membrane for a given application. Applied relative pressures are adjusted for mechanical strain up to 25%.

As would be apparent to those in the field, some features of the bioreactor are optional and most of the features exist in a variety of embodiments. In some embodiments, cells can be sourced from any mammalian species or engineered as organoids 1 from cells and/or extracellular matrix. Any organ tissue type is suitable for use in the disclosed system, compositions and methods. For example, tissues can act as surrogates for any organ, including but not limited to the heart, brain, nerve, liver, kidney, adrenal gland, stomach, pancreas, gall bladder, lung, small intestine, colon, bladder, prostate, uterus, blood, vascular, tumor, eye, and skin.

Organoid cartridge 20 containing organoid 1 is typically a cube made of a transparent solid that can be disposable or sterilizable, with at least two access ports such as doors. Suitable transparent solids include glass, and clear plastics such as polystyrene, acrylic and polycarbonate. Organoid cartridge 20 can also be any polygonal shape provided that detection/recording device 2 can detect and record the behavior of cells in organoid 1 within organoid cartridge 20. Given that the structure of organoid cartridge 20 is limited by the need to allow detection/recording device 2 to detect cell behavior, it is apparent that a variety of transparent and translucent materials may be used in constructing organoid cartridge 20. Even opaque materials are envisioned in embodiments where detection/recording device 2 is not detecting the transmission of visible light from organoid 1. Organoid cartridge 20 is also constructed to be fluid-tight, thereby allowing organoid cartridge 20 to contain cartridge media 93 to feed the cells of organoid 1. Also, a cartridge lid can provide apertures for penetration of at least one electrode or a pressure probe, *i.e.,* pressure transducer 95.

At least one organoid cartridge 20 is contained in an organoid module 10, which is formed from materials similar to the materials used for organoid cartridge 20. Organoid modules 10 are typically square or rectangular in plane view, and typically contain a top in addition to a bottom. Organoid modules 10 are sized to accommodate at least 1, 2, 3, 4, 5, 6, 8, 10, or more organoid cartridges 20. The walls, top and bottom of organoid module 10 are typically formed of a transparent solid such as glass or a clear plastic (*e.g.,* acrylic or polycarbonate), but may also be formed of translucent or opaque materials provided that detection/recording device 2 can detect, and record, cell behavior. Organoid module 10 also typically contains one or more light sources 12, and one or more mirrors 13, such as a pyramidal mirror 13. In several embodiments, there is at least one light source 12 and at least one surface of a mirror 13 for each organoid cartridge 20 contained in an organoid module 10.

Remaining components of the system include tanks, such as fresh media tank 60, mix/recycle tank 73, and additive tank 63, which are vessels for containing fluids used in the bioreactor. Such tanks can be any of a variety of dimensions and made from any of a number of materials, provided that the tanks as constructed can be used in an environment designed to minimize biological contamination, such as a sterile environment, and provided that the material used is compatible with the creation of one or more ports for fluid movement. Embodiments of the bioreactor may also involve one or more pumps, such as pump A 70, pump B 71 and pump C 72, and such pumps can be the same or different and can operate on any principle known to provide for the movement of fluids such as air and/or media through tubes. Exemplary pumps include peristaltic pumps, siphon pumps compatible with sterile environments, positive-displacement pumps such as piston-driven pumps, and non-positive-displacement pumps such as centrifugal pumps. In some embodiments, gravity is used to move fluids and no pumps are used to move, *e.g.*, media.

Organoid module 10 also can interface with various tubes to move gas, such as air, used to provide pressure, *e.g.,* inflate an organoid, which can be a balloon (*e.g.,* a 6-Fr silicon Foley catheter balloon) or to move fluid. Pressure variations sufficient to control the inflation of a balloon or to move fluid in the system are achieved at pressures compatible with the use of a wide array of tube types and not just tubing certified to handle high pressure. For example, clear, plastic, flexible tubing is suitable for use, such as Tygon^{®} tubing. Moreover, the various tubes can be combined into a single run of tubing as noted above, and such combined tubing is particularly well-suited for use with peristaltic pumps. Additionally, the tubing used in a given embodiment can vary in composition, internal diameter and external diameter. Another feature of the system are the junctions. Junctions typically are connected or attached to two or three tubes, which can vary in diameter and composition, as noted above. These junctions can be mere conduits or, more typically, are valves capable of directing the flow of fluid such as media from any attached tube to any other one or two attached tubes. Additional features and variations thereof will become apparent from the entirety of the disclosure provided herein.

In some embodiments, the organoid model has inflow and outflow fluid pathways (Figure 13A). Valves (*e.g.,* check valves, solenoid valves) control the direction of fluid movement in and out of an organoid with a cavity. In some embodiments, a single shaft or tube for inflow and outflow is contemplated (Figure 13B). A fluidic pump controls fluid flow rate into and out of the organoid. In some embodiments, unequal inflow and outflow fluid rates are used to control the amount of fluid within the organoid. Adjusting the volume within the organoid cavity results in mechanical stretch in pliable organoids. In some embodiments, stretch is applied as a step function (passive stretch) or a sigmoidal function (cyclic stretch). Mechanical stretch is considered to be a mechanotransduction signal in many organoid types. In some embodiments, a combination of mechanical and electrical stimulation presents a more robust response for therapeutic screening.

A fluidic exchange system automates routine media changes, adjusts intraluminal pressure, perfuses therapeutics during screening, and exchanges media between organoids (Figures 11 and 12). The fluidic system consists of a series microfluidic pumps, 3-way valves controlled by a digital output board, and media reservoirs. Changing the valve configuration alters the direction in which media travels. In some embodiments, fluid can be added to, or removed from, a hollow vertical mounting shaft to which an organoid is connected, thus adjusting the hydrostatic pressure. A pressure transducer 95 and signal conditioner (*e.g.,* OPP-M and LifeSens) senses the mean pressure within the organoid and communicates with the pumps via LabVIEW to adjust for a desired intraluminal pressure. Additionally, the fluidic exchange system is used for mixing and perfusion of compounds to the organoid. A solution is pumped from the additive tank and mixed with the circulating media. The compound then perfuses into organoid cartridge 20 and through organoid 1, similar to drug delivery via blood flow in humans. In some embodiments, the fluidic system of pumps and valves connects at least two organoid cartridges 20 within an organoid module 10 to permit exchange of media and/or therapeutic(s) between or among the organoids 1. Additionally, in some embodiments the fluidic exchange system between organoid cartridges 20 is powered by a biological pump in the form of an organoid 1, such as a cardiac organoid 1.

### Example 7

*Bioreactor Controls.* Custom LabVIEW code automates a large portion of the process, including both hardware and software. Each organoid module 10 is discretely controlled via a single LabVIEW-powered computer (*i.e.,* data processor 5). See Figure 16 for exemplary software flow diagrams. Therefore, multiple organoids 1 and multiple organoid modules 10 are, or can be, monitored simultaneously under different conditions (Figure 14). The LabVIEW code controls relevant hardware, such as data acquisition devices, multichannel digital output sources, valves, pumps and camera capture cards. Therefore, the code electronically controls multiple functions of the bioreactor platform or system, such as automatic drug perfusion and mixing, intraluminal pressure control, electrical stimulation, CO₂ and temperature control, and pressure transduction with synchronized image capture. The computer is outfitted with enough memory and storage space to continually capture data (*e.g.,* sufficient for at least 24 hours of continuous data collection). Image acquisition is synchronized with other acquisition modalities of the bioreactor (*e.g.,* intra-organoid pressure measurements) to enable clinically relevant endpoint measurements (*e.g.,* pressure-volume loops). Several analytical functions within the LabVIEW code enhance and simplify user functionality of the bioreactor. Particle analysis of threshold digital images quantifies real-time volume of multiple discrete organoids 1, *e.g.,* via a pyramidal mirror 13, which can be used to calculate contractile characteristics in real time for relevant organoids 1. These functions are, or can be, combined with the control of an electrical stimulator for automatic maximum capturing frequency analysis and related electrophysiological testing protocols. For example, for heart organoids 1, the LabVIEW code begins by sending a 0.5 Hz biphasic electrical stimulation pulse to the heart organoid 1 and monitors whether the organoid 1 captures the current frequency. The code automatically increases the rate of electrical stimulation until 1:1 capture is lost, where the heart organoid 1 beating frequency ceases to match the stimulation rate. Recording date, drug intervention times, electrical pacing regimens and additional information on each organoid 1 probed are saved as metadata for archiving and quality control purposes.

### Example 8

*Data Capture.* Pressure and volume data from the bioreactor are recorded simultaneously to generate pressure-volume curves in relevant contractile organoids. A high-speed digital camera (Allied Vision) acquires images up to 100 frames/second. Organoid volume is estimated by assuming an equivalent sphere with the same cross-sectional area. A single acquisition typically contains multiple contractions. To characterize an organoid for its mean contraction characteristics, MATLAB code first separates the curve into discrete contractions. The data from each contraction are then aligned and averaged (Figure 15A). The average pressure curve and average volume curve can then be plotted as the mean P-V loop (Figure 15B).

Recorded high-speed brightfield videos are analyzed (*e.g.,* optical flow) to characterize the motion pattern of the contractile organoids. Changes in contractile profile are analyzed to confirm therapeutic effects on organoid contractile performance. To handle the large amount of multidimensional data acquisition, machine learning algorithms determine key parameters that correlate with a therapeutic response and, ultimately, classify unknown therapeutics into categories of interest. Additionally, machine learning can be executed concurrently with long-term data acquisition to identify rare abnormal events and minimize data storage. For example, long-term data acquisition can be broken into a continuous series of acquisitions. Completed acquisitions are sent to the buffer to be analyzed as further acquisitions continue. Machine learning (*e.g.,* binary support vector machine) evaluates any abnormality in function from data within the buffer, such as a rare abnormal event. If an abnormality is detected, then the relevant data are permanently stored, while normal function data are discarded.

### Example 9

*Organoid Module.* In some embodiments of the bioreactor disclosed herein, the enclosure (about 25x25x15 cm) is made of a sterilizable material with a detection/recording device 2, *e.g.,* a camera, and a temperature control element 4, *e.g.,* a heating unit, attached to the roof of organoid module 10. In some embodiments, the temperature control element 4, *e.g.,* in the form of a heater, is placed within the enclosure. The vertical camera is focused onto a foursided 45-degree pyramidal mirror 13 that reflects the side profile of one or multiple organoids 1 upwards to the camera. Angled LED lights 12 evenly illuminate the side profile of each organoid 1. Access doors allow interchangeable organoid cartridges 20 to simply be inserted into the organoid module 10 for monitoring and then taken out for other experimental analyses (*e.g.,* optical mapping). After therapeutic administration to the organoid 1, the media 93 is mixed using a mixer 19 in the form of a miniature magnetic stirrer (*e.g.,* ThermoSci Micro Stirrer) that can be switched on and off using software control. Each organoid module 10 is temperature-controlled using a temperature control element 4 comprising a thermostat, heater and fan (*e.g.,* IncuKit Mini). CO₂ levels are also individually controlled at 5% for cell culture buffering. The platform's CO₂ control system comprises a single tank connected to a pressure regulator, routed to a solenoid valve manifold (*e.g.,* Takasago CTV-2-4MIC) and finally a flowmeter (Dwyer Mini-Master Flowmeter) before connection to each organoid module 10. Each valve is individually controlled via a multichannel digital output module (*e.g.,* NI-9472). A CO₂ sensor (*e.g.,* SprintIR) within the enclosure measures the CO₂ level and controls the valve to switch between open and closed states. Additional sensors (*e.g.,* O₂ sensor) are contemplated for incorporation to further control specific partial pressures within the enclosed environment.

Microtissues are not ideal for emulating human organ response as they lack key features of larger organs, such as the diffusion limitations of thicker tissues. A bioreactor that permits fluidic exchange between multiple macroscopic organoids recapitulates critical physiological and pharmacological features of the human body. The ability to measure multiple functional properties in a simplified biomimetic model of the human body provides new avenues to bridge the long-standing gap between traditional cell culture systems, *in vivo* animal models and clinical trials. In combination with somatic reprogramming of induced hPSC, the "human-body-in-a-jar" system is expected to serve as a versatile platform for next-generation drug discovery, cardiotoxicity screening, disease modeling and other ethnicity-, sex- and patient-specific applications.

### REFERENCES

1. Weng, Z., et al., A simple, cost-effective but highly efficient system for deriving ventricular cardiomyocytes from human pluripotent stem cells. Stem Cells Dev, 2014. 23(14): p. 1704-16.
2. Serrao, G.W., et al., Myocyte-depleted engineered cardiac tissues support therapeutic potential of mesenchymal stem cells. Tissue Eng Part A, 2012. 18(13-14): p. 1322-33.
3. Mannhardt, I., et al., Blinded Contractility Analysis in hiPSC-Cardiomyocytes in Engineered Heart Tissue Format: Comparison With Human Atrial Trabeculae. Toxicol Sci, 2017. 158(1): p. 164-175.
4. Mannhardt, I., et al., Human Engineered Heart Tissue: Analysis of Contractile Force. Stem Cell Reports, 2016. 7(1): p. 29-42.
5. Brinkschulte, M., et al., Plasma protein binding of perazine and amitriptyline in psychiatric patients. Eur J Clin Pharmacol, 1982. 22(4): p. 367-73.
6. Heard, K., et al., Tricyclic antidepressants directly depress human myocardial mechanical function independent of effects on the conduction system. Acad Emerg Med, 2001. 8(12): p. 1122-7.
7. Ronnevik, P.K., et al., Increased occurrence of exercise-induced silent ischemia after treatment with aspirin in patients admitted for suspected acute myocardial infarction. Int J Cardiol, 1991. 33(3): p. 413-7.
8. Piroddi, N., et al., Tension generation and relaxation in single myofibrils from human atrial and ventricular myocardium. Pflugers Arch, 2007. 454(1): p. 63-73.
9. Rasmussen, C.A., Jr., J.L. Sutko, and W.H. Barry, Effects of ryanodine and caffeine on contractility, membrane voltage, and calcium exchange in cultured heart cells. Circ Res, 1987. 60(4): p. 495-504.
10. Hansen, P.B., et al., Influence of atenolol and nifedipine on digoxin-induced inotropism in humans. Br J Clin Pharmacol, 1984. 18(6): p. 817-22.
11. Sugiyama, A., et al., Negative chronotropic and inotropic effects of class I antiarrhythmic drugs assessed in isolated canine blood-perfused sinoatrial node and papillary muscle preparations. Heart Vessels, 1999. 14(2): p. 96-103.
12. Ahonen, J., et al., Pharmacokinetic-pharmacodynamic relationship of dobutamine and heart rate, stroke volume and cardiac output in healthy volunteers. Clin Drug Investig, 2008. 28(2): p. 121-7.
13. Usta, C., et al., Comparision of the inotropic effects of levosimendan, rolipram, and dobutamine on human atrial trabeculae. J Cardiovasc Pharmacol, 2004. 44(5): p. 622-5.
14. Zhao, H., et al., Effects of dopamine on L-type Ca2+ current in single atrial and ventricular myocytes of the rat. Br J Pharmacol, 1997. 121(7): p. 1247-54.
15. Holtzman, J.L., et al., The pharmacodynamic and pharmacokinetic interaction between single doses of flecainide acetate and verapamil: effects on cardiac function and drug clearance. Clin Pharmacol Ther, 1989. 46(1): p. 26-32.
16. Johnston, A., S. Warrington, and P. Turner, Flecainide pharmacokinetics in healthy volunteers: the influence of urinary pH. Br J Clin Pharmacol, 1985. 20(4): p. 333-8.
17. Rothschild, M.A., A.H. Rothschild, and M.A. Pfeifer, The inotropic action of tolbutamide and glyburide. Clin Pharmacol Ther, 1989. 45(6): p. 642-9.
18. Hasenfuss, G., et al., Influence of isoproterenol on contractile protein function, excitation-contraction coupling, and energy turnover of isolated nonfailing human myocardium. J Mol Cell Cardiol, 1994. 26(11): p. 1461-9.
19. Sheu, S.S. and W.J. Lederer, Lidocaine's negative inotropic and antiarrhythmic actions. Dependence on shortening of action potential duration and reduction of intracellular sodium activity. Circ Res, 1985. 57(4): p. 578-90.
20. Nicolosi, G.L., et al., The prognostic value of predischarge quantitative two-dimensional echocardiographic measurements and the effects of early lisinopril treatment on left ventricular structure and function after acute myocardial infarction in the GISSI-3 Trial. Gruppo Italiano per lo Studio della Sopravvivenza nell'Infarto Miocardico. Eur Heart J, 1996. 17(11): p. 1646-56.
21. Sarsero, D., et al., Human vascular to cardiac tissue selectivity of L- and T-type calcium channel antagonists. Br J Pharmacol, 1998. 125(1): p. 109-19.
22. Baruch, L., et al., Pharmacodynamic effects of milrinone with and without a bolus loading infusion. Am Heart J, 2001. 141(2): p. 266-73.
23. Rumiantsev, D.O., et al., Serum binding of nifedipine and verapamil in patients with ischaemic heart disease on monotherapy. Br J Clin Pharmacol, 1989. 28(3): p. 357-61.
24. Leenen, F.H., et al., Epinephrine and left ventricular function in humans: effects of beta-1 vs nonselective beta-blockade. Clin Pharmacol Ther, 1988. 43(5): p. 519-28.
25. Kool, M., et al., Does lowering of cholesterol levels influence functional properties of large arteries? Eur J Clin Pharmacol, 1995. 48(3-4): p. 217-23.
26. Ochs, H.R., et al., Single and multiple dose pharmacokinetics of oral quinidine sulfate and gluconate. Am J Cardiol, 1978. 41(4): p. 770-7.
27. Johnson, D.B., et al., Angiotensin-converting enzyme inhibitor therapy affects left ventricular mass in patients with ejection fraction > 40% after acute myocardial infarction. J Am Coll Cardiol, 1997. 29(1): p. 49-54.
28. Turnbull, I.C., et al., Advancing functional engineered cardiac tissues toward a preclinical model of human myocardium. FASEB J, 2014. 28(2): p. 644-54.
29. Lee, E. J., et al. Engineered cardiac organoid chambers: toward a functional biological model ventricle. Tissue Eng Part A. 2008;14:215-25.

It is to be understood that while the claimed subject matter has been described in conjunction with the detailed description thereof, the foregoing description is intended to illustrate and not limit the scope of that claimed subject matter, which is defined by the scope of the appended claims.

## Claims

1. A tissue monitoring system comprising
(a) At least one organoid module comprising a plurality of organoid cartridges, wherein each organoid cartridge comprises a media inlet, a media outlet, and at least one wall compatible with an external detection device, wherein a plurality of the organoid cartridges each comprise a biological material comprising at least one human cell, wherein the cell is a human embryonic stem cell, a human adult stem cell, a human induced pluripotent stem cell, a cell derived from a human tissue, or a progenitor cell of a human tissue;
(b) a mirror arrangement for simultaneous monitoring of any biological development of the biological material in each of at least two organoid cartridges; and
(c) a detection device for observing the monitored biological development of the biological material in each of at least two organoid cartridges.

2. The system of claim 1, wherein the mirror arrangement comprises at least one pyramidal mirror.

3. The system of claim 1 or 2, wherein the biological material is at least one tissue or at least one organoid, wherein the organoid preferably is a heart, a brain, a nerve, a liver, a kidney, an adrenal gland, a stomach, a pancreas, a gall bladder, a lung, a small intestine, a colon, a bladder, a prostate, a uterus, a tumor, an eye, skin, blood, or a vascular organoid, and wherein the organoid most preferably is a heart organoid.

4. The system of any one of claims 1 to 3, further comprising at least one of:
(a) an electrode in adjustable relation to the cell, tissue, or organoid in at least one organoid cartridge;
(b) a temperature control element, a light source, a module access port, or any combination thereof;
(c) a data processor in electronic communication with the detection device, a temperature control element, a light source, a module access port or any combination thereof;
(d) a tissue comprising at least one human cell;
(e) a monitor;
(f) a plurality of organoid modules;
(g) an interconnected fluid exchange network, wherein the network comprises a plurality of fluid lines, a plurality of valves, at least one pump, and at least one fluid tank;
(h) a gas pressure controller, wherein the gas pressure controller preferably controls the concentration of at least one of O₂ and CO₂ in at least one module or in one or more organoid cartridges;
(i) a plurality of module access ports; and
(j) a drug perfusion apparatus for delivery of a therapeutic to the cell, tissue, or organoid.

5. The system of any one of claims 1 to 4, wherein the detection device is a recording device, wherein the recording device preferably is a digital camera, a high-speed camera, at least one pressure transducer, or a combination of a digital camera and at least one pressure transducer.

6. The system of claim 4, comprising the interconnected fluid exchange network and further comprising a port for introduction of a compound, wherein the compound preferably is a candidate therapeutic, drug, a viral vector, conditioned media, extracellular vesicles, additional cells, or any combination thereof.

7. The system of claim 4, comprising the interconnected fluid exchange network, wherein
- the interconnected fluid exchange network comprises fluid communication between at least two organoid cartridges, or
- the interconnected fluid exchange network provides a partially common fluid delivery path for at least two organoid cartridges, a partially common fluid removal path for at least two organoid cartridges, or both a partially common fluid delivery path and a partially common fluid removal path for at least two organoid cartridges, or
- the fluid is media, or
- the fluid exchange network provides automated media exchange.

8. A method for assaying a compound for bioactivity comprising administering a compound to the cell, tissue, or organoid in the system of any one of claims 1 to 7 and monitoring the response of the cell, tissue or organoid to the compound.

9. The method of claim 8, wherein the compound is a drug, a viral vector, conditioned media, extracellular vesicles, additional cells, or any combination thereof, wherein the bioactivity is modulation of a function of the cell, tissue, or organoid, thereby identifying the compound as a therapeutic for treatment of a disorder of the organ cognate of the cell, tissue, or organoid, and/or wherein the assay measures the toxicity of the compound.

10. A system for screening a compound for inotropism comprising:
(a) a first-stage screening apparatus comprising:
(i) a biocompatible gel comprising a plurality of cardiomyocytes;
(ii) a biocompatible support apparatus for suspending the biocompatible gel, wherein the biocompatible gel and biocompatible support apparatus form a cardiac tissue strip, the cardiac tissue strip is preferably 26.5 mm in length by 16 mm in width by 6 mm in height;
(iii) a detection device for detecting movement of the biocompatible gel; and
(iv) an electrical power source for applying an electrical pacing stimulus to the biocompatible gel; and
(b) a second-stage screening apparatus comprising the tissue monitoring system of any one of claims 1 to 7, and wherein at least one organoid cartridge comprises cardiac biological material.

11. The system of claim 10, further comprising a media mixer, wherein the media mixer preferably is a magnetic stirring apparatus or a turntable.

12. The system according to claim 10 or 11, wherein the cardiomyocytes are present at a concentration of at least 10⁶ cells/ml and/or are human cardiomyocytes, which human cardiomyocytes preferably are human ventricular cardiomyocytes or derived from at least one human pluripotent stem cell.

13. The system according to claim any one of claim 10 to 12, wherein the biocompatible gel
- comprises matrigel, wherein the matrigel preferably is present at a concentration of at least 0.5 mg/ml; and optionally
- further comprises collagen, wherein the collagen preferably is type I human collagen and/or is present at a concentration of at least 1 mg/ml.

14. The system according to any one of claims 10 to 13, wherein the support apparatus is at least two vertical support members, wherein the vertical support members are preferably made of polydimethylsiloxane.

15. The system according to claim 14, wherein there are two vertical support members, and wherein the two vertical support members are preferably approximately circular in cross-section with a diameter of 0.5 mm.

## Patentansprüche

1. System zur Überwachung von Gewebe, das Folgendes umfasst:
(a) mindestens ein Organoid-Modul, das eine Vielzahl von Organoid-Kartuschen umfasst, wobei jede Organoid-Kartusche einen Medieneinlass, einen Medienauslass und mindestens eine Wand umfasst, die mit einer externen Erfassungsvorrichtung kompatibel ist, wobei eine Vielzahl der Organoid-Kartuschen jeweils ein biologisches Material umfasst, das mindestens eine menschliche Zelle umfasst, wobei die Zelle eine menschliche embryonale Stammzelle, eine menschliche adulte Stammzelle, eine menschliche induzierte pluripotente Stammzelle, eine von einem menschlichen Gewebe abgeleitete Zelle oder eine Progenitorzelle eines menschlichen Gewebes ist;
(b) eine Spiegelanordnung zur gleichzeitigen Überwachung einer biologischer Entwicklung des biologischen Materials in jeder der mindestens zwei Organoid-Kartuschen; und
(c) eine Erfassungsvorrichtung zum Beobachten der überwachten biologischen Entwicklung des biologischen Materials in jeder der mindestens zwei Organoid-Kartuschen.

2. System nach Anspruch 1, wobei die Spiegelanordnung mindestens einen Pyramidenspiegel umfasst.

3. System nach Anspruch 1 oder 2, wobei das biologische Material mindestens ein Gewebe oder mindestens ein Organoid ist, wobei das Organoid vorzugsweise ein Herz, ein Gehirn, ein Nerv, eine Leber, eine Niere, eine Nebenniere, ein Magen, eine Bauchspeicheldrüse, eine Gallenblase, eine Lunge, ein Dünndarm, ein Dickdarm, eine Blase, eine Prostata, ein Uterus, ein Tumor, ein Auge, eine Haut, Blut oder ein vaskuläres Organoid ist, und wobei das Organoid vorzugsweise ein Herzorganoid ist.

4. System nach einem der Ansprüche 1 bis 3, das des Weiteren mindestens eines aus Folgendem umfasst:
(a) eine Elektrode in einstellbarer Beziehung zu der Zelle, dem Gewebe oder dem Organoid in mindestens einer Organoid-Kartusche;
(b) ein Temperatursteuerungselement, eine Lichtquelle, einen Modulzugangsanschluss oder eine beliebige Kombination davon;
(c) einen Datenprozessor in elektronischer Kommunikation mit der Erfassungsvorrichtung, einem Temperatursteuerungselement, einer Lichtquelle, einem Modulzugangsanschluss oder einer beliebigen Kombination davon;
(d) ein Gewebe, das mindestens eine menschliche Zelle umfasst;
(e) einen Monitor;
(f) eine Vielzahl von Organoid-Modulen;
(g) ein miteinander verbundenes Flüssigkeitsaustauschnetz, wobei das Netz eine Vielzahl von Flüssigkeitsleitungen, eine Vielzahl von Ventilen, mindestens eine Pumpe und mindestens einen Flüssigkeitstank umfasst;
(h) einen Gasdruckregler, wobei der Gasdruckregler vorzugsweise die Konzentration von mindestens einem aus O₂ und CO₂ in mindestens einem Modul oder in einer oder mehreren Organoid-Kartuschen regelt;
(i) eine Vielzahl von Modulzugangsanschlüssen; und
(j) eine Einrichtung zur Perfusion von Medikamenten für die Zufuhr eines Therapeutikums zu der Zelle, dem Gewebe oder dem Organoid.

5. System nach einem der Ansprüche 1 bis 4, wobei die Erfassungsvorrichtung eine Aufzeichnungsvorrichtung ist, wobei die Aufzeichnungsvorrichtung vorzugsweise eine Digitalkamera, eine Hochgeschwindigkeitskamera, mindestens ein Druckwandler oder eine Kombination aus einer Digitalkamera und mindestens einem Druckwandler ist.

6. System nach Anspruch 4, das das miteinander verbundene Flüssigkeitsaustauschnetz umfasst und das des Weiteren einen Anschluss zur Einführung einer Verbindung umfasst, wobei die Verbindung vorzugsweise ein potentielles Therapeutikum, ein Medikament, ein viraler Vektor, konditionierte Medien, extrazelluläre Vesikel, zusätzliche Zellen oder eine beliebige Kombination davon ist.

7. System nach Anspruch 4, welches das miteinander verbundene Flüssigkeitsaustauschnetz umfasst, wobei
- das miteinander verbundene Flüssigkeitsaustauschnetz eine Flüssigkeitsverbindung zwischen mindestens zwei Organoid-Kartuschen umfasst, oder
- das miteinander verbundene Flüssigkeitsaustauschnetz einen teilweise gemeinsamen Flüssigkeitszufuhrpfad für mindestens zwei Organoid-Kartuschen, einen teilweise gemeinsamen Flüssigkeitsabfuhrpfad für mindestens zwei Organoid-Kartuschen oder sowohl einen teilweise gemeinsamen Flüssigkeitszufuhrpfad als auch einen teilweise gemeinsamen Flüssigkeitsabfuhrpfad für mindestens zwei Organoid-Kartuschen bereitstellt, oder
- die Flüssigkeit ein Medium ist, oder
- das Flüssigkeitsaustauschnetz einen automatischen Medienaustausch ermöglicht.

8. Verfahren zur Untersuchung einer Verbindung auf Bioaktivität, umfassend die Verabreichung einer Verbindung an die Zelle, das Gewebe oder das Organoid im System nach einem der Ansprüche 1 bis 7 und das Überwachen der Reaktion der Zelle, des Gewebes oder des Organoids auf die Verbindung.

9. Verfahren nach Anspruch 8, wobei es sich bei der Verbindung um ein Arzneimittel, einen viralen Vektor, konditionierte Medien, extrazelluläre Vesikel, zusätzliche Zellen oder eine beliebige Kombination davon handelt, wobei die Bioaktivität die Modulation einer Funktion der Zelle, des Gewebes oder des Organoids ist, wodurch die Verbindung als Therapeutikum zur Behandlung einer Störung des mit der Zelle, dem Gewebe oder dem Organoid verwandten Organs identifiziert wird, und/oder wobei die Untersuchung die Toxizität der Verbindung misst.

10. System zum Screening einer Verbindung auf Inotropie, das umfasst:
(a) eine Screeningvorrichtung der ersten Stufe, die Folgendes umfasst:
(i) ein biokompatibles Gel, das eine Vielzahl von Kardiomyozyten enthält;
(ii) eine biokompatible Stützvorrichtung zum Aufhängen des biokompatiblen Gels, wobei das biokompatible Gel und die biokompatible Stützvorrichtung einen Herzgewebestreifen bilden, wobei der Herzgewebestreifen vorzugsweise 26,5 mm lang, 16 mm breit und 6 mm hoch ist;
(iii) eine Erfassungsvorrichtung zum Erfassen der Bewegung des biokompatiblen Gels; und
(iv) eine elektrische Stromquelle zum Anlegen eines elektrischen Stimulationsimpulses an das biokompatible Gel; und
(b) eine Screeningvorrichtung der zweiten Stufe, die das System zur Überwachung von Gewebe nach einem der Ansprüche 1 bis 7 umfasst, und wobei mindestens eine Organoid-Kartusche kardiales biologisches Material umfasst.

11. System nach Anspruch 10, das des Weiteren einen Medienmischer umfasst, wobei der Medienmischer vorzugsweise eine magnetische Rühreinrichtung oder ein Drehtisch ist.

12. System nach Anspruch 10 oder 11, wobei die Kardiomyozyten in einer Konzentration von mindestens 10⁶ Zellen/ml vorliegen und/oder menschliche Kardiomyozyten sind, wobei die menschlichen Kardiomyozyten vorzugsweise menschliche ventrikuläre Kardiomyozyten sind oder von mindestens einer menschlichen pluripotenten Stammzelle stammen.

13. System nach einem der Ansprüche 10 bis 12, wobei das biokompatible Gel
- Matrigel umfasst, wobei das Matrigel vorzugsweise in einer Konzentration von mindestens 0,5 mg/ml vorhanden ist; und optional
- des Weiteren Kollagen umfasst, wobei das Kollagen vorzugsweise menschliches Kollagen vom Typ I ist und/oder in einer Konzentration von mindestens 1 mg/ml vorliegt.

14. System nach einem der Ansprüche 10 bis 13, wobei die Stützvorrichtung aus mindestens zwei vertikalen Stützelementen besteht, wobei die vertikalen Stützelemente vorzugsweise aus Polydimethylsiloxan hergestellt sind.

15. System nach Anspruch 14, wobei zwei vertikale Stützelemente vorhanden sind und wobei die zwei vertikalen Stützelemente vorzugsweise annähernd kreisförmig im Querschnitt mit einem Durchmesser von 0,5 mm sind.

## Revendications

1. Système de surveillance tissulaire comprenant
a) au moins un module organoïde comprenant une pluralité de cartouches d'organoïde, dans lequel chaque cartouche d'organoïde comprend une entrée de milieu, une sortie de milieu, et au moins une paroi compatible avec un dispositif de détection externe, dans lequel une pluralité des cartouches d'organoïde comprennent chacune un matériel biologique comprenant au moins une cellule humaine, la cellule étant une cellule souche embryonnaire humaine, une cellule souche adulte humaine, une cellule souche pluripotente induite humaine, une cellule dérivée d'un tissu humain, ou une cellule progénitrice d'un tissu humain ;
b) un équipement de miroir pour la surveillance simultanée de tout développement biologique du matériel biologique dans chacune d'au moins deux cartouches d'organoïde ; et
c) un dispositif de détection pour observer le développement biologique surveillé du matériel biologique dans chacune d'au moins deux cartouches d'organoïde.

2. Système selon la revendication 1, dans lequel l'équipement de miroir comprend au moins un miroir pyramidal.

3. Système selon la revendication 1 ou 2, dans lequel le matériel biologique est au moins un tissu ou au moins un organoïde, dans lequel l'organoïde est de préférence un cœur, un cerveau, un nerf, un foie, un rein, une glande surrénale, un estomac, un pancréas, une vésicule biliaire, un poumon, un intestin grêle, un côlon, une vessie, une prostate, un utérus, une tumeur, un œil, de la peau, du sang, ou un organoïde vasculaire, et dans lequel l'organoïde est de manière la plus préférée un organoïde cardiaque.

4. Système selon l'une quelconque des revendications 1 à 3, comprenant en outre au moins l'un parmi :
a) une électrode en relation réglable avec la cellule, le tissu ou l'organoïde dans au moins une cartouche d'organoïde ;
b) un élément de régulation de température, une source lumineuse, un orifice d'accès à un module, ou toute combinaison de ceux-ci ;
c) un processeur de données en communication électronique avec le dispositif de détection, un élément de régulation de température, une source lumineuse, un orifice d'accès à un module, ou toute combinaison de ceux-ci ;
d) un tissu comprenant au moins une cellule humaine ;
e) un moniteur ;
f) une pluralité de modules organoïdes ;
g) un réseau d'échange de fluide interconnecté, dans lequel le réseau comprend une pluralité de conduites de fluide, une pluralité de vannes, au moins une pompe, et au moins un réservoir de fluide ;
h) un régulateur de pression de gaz, dans lequel le régulateur de pression de gaz régule de préférence la concentration d'au moins l'un parmi O₂ et CO₂ dans au moins un module ou dans une ou plusieurs cartouches d'organoïde ;
(i) une pluralité d'orifices d'accès à un module ; et
(i) un appareil de perfusion médicamenteuse pour l'administration d'un agent thérapeutique à la cellule, au tissu ou à l'organoïde.

5. Système selon l'une quelconque des revendications 1 à 4, dans lequel le dispositif de détection est un dispositif d'enregistrement, dans lequel le dispositif d'enregistrement est de préférence un appareil de prise de vues numérique, un appareil de prise de vues à grande vitesse, au moins un transducteur de pression, ou une combinaison d'un appareil de prise de vues numérique et d'au moins un transducteur de pression.

6. Système selon la revendication 4, comprenant le réseau d'échange de fluide interconnecté et comprenant en outre un orifice pour l'introduction d'un composé, dans lequel le composé est de préférence un candidat thérapeutique, un médicament, un vecteur viral, un milieu conditionné, des vésicules extracellulaires, des cellules supplémentaires, ou toute combinaison de ceux-ci.

7. Système selon la revendication 4, comprenant le réseau d'échange de fluide interconnecté, dans lequel
- le réseau d'échange de fluide interconnecté comprend une communication fluidique entre au moins deux cartouches d'organoïde, ou
- le réseau d'échange de fluide interconnecté fournit un trajet d'administration de fluide partiellement commun pour au moins deux cartouches d'organoïde, un trajet d'évacuation de fluide partiellement commun pour au moins deux cartouches d'organoïde, ou à la fois un trajet d'administration de fluide partiellement commun et un trajet d'évacuation de fluide partiellement commun pour au moins deux cartouches d'organoïde, ou
- le fluide est un milieu, ou
- le réseau d'échange de fluide permet un échange automatisé de milieu.

8. Procédé pour évaluer un composé quant à la bio-activité, comprenant l'administration d'un composé à la cellule, au tissu, ou à l'organoïde dans le système selon l'une quelconque des revendications 1 à 7 et la surveillance de la réponse de la cellule, du tissu ou de l'organoïde au composé.

9. Procédé selon la revendication 8, dans lequel le composé est un médicament, un vecteur viral, un milieu conditionné, des vésicules extracellulaires, des cellules supplémentaires, ou toute combinaison de ceux-ci, dans lequel la bioactivité est une modulation d'une fonction de la cellule, du tissu, ou de l'organoïde, identifiant ainsi le composé comme un agent thérapeutique pour le traitement d'un trouble de l'organe parent de la cellule, du tissu, ou de l'organoïde, et/ou dans lequel l'évaluation mesure la toxicité du composé.

10. Système de criblage d'un composé quant à l'inotropisme comprenant :
a) un appareil de criblage de premier étage comprenant :
(i) un gel biocompatible comprenant une pluralité de cardiomyocytes ;
(ii) un appareil de support biocompatible pour mettre en suspension le gel biocompatible, dans lequel le gel biocompatible et l'appareil de support biocompatible forment une bande de tissu cardiaque, la bande de tissu cardiaque présentant de préférence 26,5 mm de longueur sur 16 mm de largeur sur 6 mm de hauteur ;
(iii) un dispositif de détection pour détecter un mouvement du gel biocompatible ; et
(iv) une source d'énergie électrique pour l'application d'un stimulus de stimulation électrique au gel biocompatible ; et
(b) un appareil de criblage de second étage comprenant le système de surveillance tissulaire selon l'une quelconque des revendications 1 à 7, et dans lequel au moins une cartouche d'organoïde comprend un matériel biologique cardiaque.

11. Système selon la revendication 10, comprenant en outre un mélangeur de milieu, dans lequel le mélangeur de milieu est de préférence un appareil d'agitation magnétique ou un plateau tournant.

12. Système selon la revendication 10 ou 11, dans lequel les cardiomyocytes sont présents à une concentration d'au moins 10⁶ cellules/ml et/ou sont des cardiomyocytes humains, lesquels cardiomyocytes humains sont de préférence des cardiomyocytes ventriculaires humains ou dérivés d'au moins une cellule souche pluripotente humaine.

13. Système selon l'une quelconque des revendications 10 à 12, dans lequel le gel biocompatible
- comprend du matrigel, dans lequel le matrigel est de préférence présent à une concentration d'au moins 0,5 mg/ml ; et facultativement
- comprend en outre du collagène, dans lequel le collagène est de préférence du collagène humain de type I et/ou est présent à une concentration d'au moins 1 mg/ml.

14. Système selon l'une quelconque des revendications 10 à 13, dans lequel l'appareil de support est constitué d'au moins deux éléments de support verticaux, dans lequel les éléments de support verticaux sont de préférence en polydiméthylsiloxane.

15. Système selon la revendication 14, dans lequel deux éléments de support verticaux sont présents, et dans lequel les deux éléments de support verticaux ont de préférence une coupe transversale approximativement circulaire avec un diamètre de 0,5 mm.
